# EUROPEAN PATENT SPECIFICATION

(11) **EP 1 137 436 B1**
(45) Date of publication and mention of the grant of the patent: **04.06.2008**
(21) Application number: 99965991.5
(22) Date of filing: 03.12.1999
(51) Int. Cl.: A61K 39/395, C07K 14/53

(54) **Stimulation of T-cells against self antigens using CTLA-4 blocking agents**
Stimulierung von T-Zellen gegen Selbstantigene unter Verwendung von CTLA-4 inhibierenden Wirkstoffen
Stimulation de lymphocytes T contre des autoantigènes au moyen d'agents bloquants CTLA-4

(30) Priority: 03.12.1998 US 110761 P
(43) Date of publication of application: 04.10.2001
(73) Proprietor: The Regents of the University of California, Berkeley, California 94720-1620 (US)
(72) Inventor: ALLISON, James, P., Berkeley, CA 94707 (US); HURWITZ, Arthur, A., Manlius, NY 13104 (US); VANELSAS, Andrea, NL-1625 EG Hoorn (NL)
(74) Representative: Harrison Goddard Foote
(86) International application number: PCT/US1999/028739
(87) International publication number: WO 2000/032231

(56) References cited:
- WO-A-90/05541
- WO-A-97/20574
- WO-A-98/42752
- WO-A2-01/14424
- A. HURWITZ ET AL.: "CTLA-4 blockade synergizes with tumor-derived granulocyte-macrophage colony-stimulating factor for treatment of an experimental mammary carcinoma." PROCEEDINGS OF THE NATIONAL ACADEMY OF SCIENCES OF THE U.S.A, vol. 95, no. 17, 18 August 1998 (1998-08-18), pages 10067-10071, XP002133809 Washington, DC, USA
- VAN ELSAS ET AL.: "Blockade of CTLA-4 combined with a GM-CSF vaccine cures mice from the highly tumorigenic, poorly immunogenic melanoma B16-BL6, and induces depigmentation." THE FASEB JOURNAL, vol. 12, no. 5, 20 March 1998 (1998-03-20), page A908 XP002133810 Bethesda, MD, USA
- C. THOMPSON ET AL.: "Emerging role of CTLA-4 as an immune attenuator." IMMUNITY, vol. 7, no. 4, October 1997 (1997-10), pages 445-450, XP000891379 Cambridge, MA, USA cited in the application
- E. CEPERO ET AL.: "Potent inhibition of CTLA-4 expression by an anti-CTLA-4 ribozyme." BIOCHEMICAL AND BIOPHYSICAL RESEARCH COMMUNICATIONS, vol. 247, no. 3, 29 June 1998 (1998-06-29), pages 838-843, XP002133811 Duluth, MN, USA
- VAN ELSAS A. ET AL J.EXP.MED. vol. 190, no. 3, August 1999, pages 355 - 366, XP002966969

## Description

### Introduction

Putting immunotherapy into practice is a highly desired goal in the treatment of human disease. It promises a specificity of action that is rarely found with the use of conventional drugs. The basis for immunotherapy is the manipulation of the immune response, particularly the responses of T cells. T cells possess complex and subtle systems for controlling their interactions, utilizing numerous receptors and soluble factors for the process. The effect that any particular signal will have on the immune response may vary, depending on the factors, receptors and counter-receptors that are involved.

Full activation of T cells requires not only an antigen-specific signal through the T cell antigen receptor (TCR), but also additional signaling through co-stimulatory surface molecules such as CD28. The ligands for CD28 are the B7-1 (CD80) and B7-2 (CD86) proteins, which are expressed on antigen-presenting cells such as dendritic cells, activated B cells or monocytes. Stimulation ofT cells by antigen in the absence of such co-stimulatory signals can result in unproductive T cell stimulation or T cell tolerance. The lack of expression of B7 by tumor cells, for example, is one factor that can contribute to their failure to elicit productive immune responses.

The pathways for down-regulating responses are as important as those required for activation. Thymic education leading to peripheral T cell tolerance is one mechanism for preventing an immune response to a particular antigen. Other mechanisms, such as secretion of suppressive cytokines, are also known. More recently, CTLA-4 was identified as a second T cell counter-receptor for B7, and has now been shown to play a critical role in attenuating T cell responses. Thompson and Allison, Immunity 7:445-450 (1997).

Non-immunogenic or poorly-immunogenic tumors present special challenges. Absent or ineffective altered antigens or viral antigens prevent the activation of an antigen-specific T cell response, allowing the tumor to grow unimpeded. Strategies for mounting a cytotoxic immune response to these types of tumor cells are also complicated by the body's natural immune tolerance to self antigens that are present in both normal and tumor cells. Overcoming immune tolerance may therefore be necessary in order to mount an effective cytotoxic T cell response. To date, however, a safe and effective method for breaking immune tolerance and stimulating autoreactive T cells has yet to be devised.

It would be advantageous if, in the treatment of infections and tumors, one could activate a strong cellular immune response through the manipulation of receptors involved in co-stimulation. A further advantage would be gained if one could break immune tolerance for a desired self antigen.

The use of B7 protein in mediating anti-tumor immunity is described in Chen et al. (1992) Cell 71:1093-1102 and Townsend and Allison (1993) Science 259:368. Schwartz (1992) Cell 71:1065 reviews the role of CD28, CTLA-4 and B7 in IL-2 production and immunotherapy. Harding et al. (1994) Nature 356:607-609 demonstrates that CD28 mediated signaling co-stimulates murine T cells and prevents the induction of anergy in T cell clones.

CTLA-4 is a T cell surface molecule that was originally identified by differential screening of a murine cytolytic T cell cDNA library, Brunet el al. (1987) Nature 328:267-270. The role of CTLA-4 as a second receptor for B7 is discussed in Linsley et al. (1991) J. Exp. Med. 174:561-569. Freeman et al. (1993) Science 262:907-909 discusses CTLA-4 in B7 deficient mice. Ligands for CTLA-4 are described in Lenschow et al. (1993) P.N.A.S. 90:11054-11058.

Linsley et al. (1992) Science 257:792-795 describes inununosuppression *in vivo* by a soluble form of CTLA-4. Lenschow et al. (1992) Science 257:789-792 discusses long term survival of pancreatic islet grafts induced by CTLA-4Ig. It is suggested in Walunas et al. (1994) Immunity 1:405-413, that CTLA-4 can function as a negative regulator ofT cell activation. Thompson and Allison, Immunity 7:445-450 (1997) reviews the accumulating data suggesting that CTLA-4 serves to attenuate T cell responses.

### Summary of the Invention

The invention relates to compositions for stimulating T cells to respond to self antigens, through a blockade of CTLA-4 signaling. CTLA-4 blocking agents are combined with self antigen preparations and optionally additional immune response stimulating agents in costimulation strategies to break immune tolerance and stimulate an enhanced T-cell response against the selfantigen. This enhanced response is useful for the treatment of non-immunogenic and poorly-immunogenic tumors, as well as other medical conditions requiring selective tissue ablation.

The invention provides use of an anti-CLA-4 antibody or fragment thereof and a self-antigen preparation for the manufacture of a medicament for treating established metastases.

In one embodiment of the invention, a CTLA-4 blocking agent is combined with a self antigen preparation comprising a tumor cell vaccine for the tumor of interest. In a particularly preferred embodiment, the tumor cell vaccine comprises irradiated tumor cells transduced to express cytokines such as granulocyte/macrophage colony-stimulating factor (GM-CSF), to enhance cross-priming ofT cells by antigen presenting cells (APCs). Alternatively, purified self antigen or a mixture of self antigens may be combined with CTLA-4 blocking agents, either alone or in combination with other immune response stimulating agents such as adjuvants or dendritic cells.

### Brief Description of the Drawings

Figure 1A is a graph illustrating the *in vivo* growth of the tumor cell line V51Blim10 in the presence or absence of antibodies directed against CTLA-4 or CD28. Figure 1B is a graph illustrating the average tumor size in mice injected with 2 x 10⁶ V51Blim10 cells and antibodies. Figure 1C is a graph illustrating individual tumor growth size in mice injected with V51Blim10 cells.
Figure 2 is a graph showing the *in vivo* growth of B7-51BLim10 tumors in the presence or absence of antibodies directed against CTLA-4 or CD28.
Figure 3 shows the rejection of wild-type colon carcinoma cells by mice previously treated with V51BLim10 cells and anti-CTLA-4 antibody.
Figure 4 shows the growth of established tumors after treatment with anti-CTLA-4 antibody.
Figure 5 shows the growth of the murine fibrosarcoma SA1N in the absence or presence of anti-CTLA-4 antibodies.
Figures 6A to 6E illustrate the adjuvant effect of anti-CTLA-4 antibodies in the response of T cells to peptide antigens.
Figures 7A to 7F illustrate the effect of CTLA-4 blockade on class switching.
Figure 8 shows the effect of delaying the CTLA-4 blockade on a fibrosarcoma.
Figure 9 shows the effect of treating a mammary carcinoma with anti-CTLA-4 alone, GM-CSF transduced cells alone or a combination thereof.
Figures 10A and 10B demonstrate the effect of delayed CTLA-4 blockade on a renal carcinoma.
Figure 11 shows the effect of CTLA-4 blockade treatment alone or in combination with immunization with irradiated B 16 tumor cells on B16 tumors.
Figure 12 shows the effect of combining the CTLA-4 blockade with irradiated B16 cells and/or cytokine treatment.
Figures 13A and 13B show the effect of CTLA-4 blockade treatment alone or in combination with irradiated B16-BL6 cells transduced with GM-CSF on B16-BL6 tumors.
Figure 14 demonstrates IFNγ production by B 16-specific T cells induced *in vivo*.
Figure 15 shows the survival rate of mice bearing B 16-F 10 lung metastases when treated with CTLA-4 blockade and F 10/GM vaccine.
Figure 16A shows that TRAMP mice treated with TRAMP-C vaccines and anti-CTLA-4 have a lower tumor incidence than control-treated animals.
Figure 16B shows tumor incidence as a function of age at the time of treatment, demonstrating a significant reduction in tumor incidence in mice treated at 14 weeks of age but not in mice treated at 16 weeks of age.
Figure 17A demonstrates the reduction in severity of prostatic lesions in TRAMP mice treated with TRAMP-C vaccines and anti-CTLA-4.
Figure 17B shows that TRAMP mice treated with GMTRAMP-C1/C2 and anti-CTLA-4 had a reduction in tumor grade when treated at 14 weeks of age but not when treated at 16 weeks.
Figure 18 shows IFN-γ production after peptide stimulation *in vivo.*
Figure 19 shows B16 melanoma tumor growth in peptide-immunized mice with and without CTLA-4 blockade.

### Database References for Nucleotide and Amino Acid Sequences

The complete cDNA sequence of human CTLA-4 has the Genbank accession number L15006. The region of amino acids 1-37 is the leader peptide; 38-161 is the extracellular V-like domain; 162-187 is the transmembrane domain; and 188-223 is the cytoplasmic domain. Variants of the nucleotide sequence have been reported, including a G to A transition at position 49, a C to T transition at position 272, and an A to G transition at position 439. The complete DNA sequence of mouse CTLA-4 has the EMBL accession number X05719 (Brunet et al. (1987) Nature 328:267-270). The region of amino acids 1-35 is the leader peptide.

The complete DNA sequence of human B7-1 (CD80) has the Genbank accession number X60958; the accession number for the mouse sequence is X60958; the accession number for the rat sequence is U05593. The complete cDNA sequence of human B7-2 (CD86) has the Genbank accession number L25259; the accession number for the mouse sequence is L25606.

The genes encoding CD28 have been extensively characterized. The chicken mRNA sequence has the Genbank accession number X67915. The rat mRNA sequence has the Genbank accession numberX55288. The human mRNA sequence has the Genbank accession number J02988. The mouse mRNA sequence has the Genbank accession number M34536.

### Detailed Description of the Invention

The invention relates to compositions for stimulating a T cell response to self antigens. CTLA-4 blocking agents namely an anti-CLA-4 antibody or fragment thereof, are employed in costimulation strategies to activate existing autoreactive T cells and/or naive T cells to respond to self antigens, selectively abrogating immune tolerance. A self antigen preparation is administered with the subject blocking agents to help stimulate an autoreactive peripheral T cell response. Preferred self antigen preparations include purified protein, lysates from tumor cells, or tumor vaccines comprising irradiated tumor cells. Of particular interest are tumor vaccines transduced with genes of interest, such as genes encoding for cytokines that stimulate antigen presenting cells, e.g. granulocyre-macrophage colony stimulating factor (GM-CSF), macrophage colony stimulating factor (M-CSF), or granulocyte colony stimulating factor (G-CSF). Additional and alternative costimulation strategies are described in more detail herein. The subject invention is useful in the treatment of poorly-immunogenic and non-immunogenic tumors, as well as in tissue ablation techniques in general.

The therapy releases T cells from inhibitory signals mediated through CTLA-4. It is thought that CTLA-4 engagement down-regulates T cell responses by raising the threshold of signals needed for effective T cell activation, or may also play a role in terminating ongoing T cell responses. The T cell response to antigen and co-stimulatory CD28 signaling is thereby up-regulated in the presence of CTLA-4 blocking agents. When employed in an appropriate costimulation strategy, CTLA-4 blockade stimulates low-avidity autoreactive T cells and/or naive T cells to respond to the self antigen. The subject methods do not promote a generalized proliferation of unstimulated T cells.

According to the invention, a subject CTLA-4 blocking agent, i.e. an anti-CLA-4 antibody or fragment thereof, is used for the manufacture of a medicament for treating established metastases.

The medicaments are useful where there is an inadequate T cell mediated response to an antigenic stimulus.

Situations characterized by deficient host T cell response to antigen include tumors. Administration of the subject CTLA-4 blockers to such hosts specifically changes the phenotype of activated T cells, resulting in increased response to antigen mediated activation. Treatment of primates, more particularly humans is of interest, but other mammals may also benefit from treatment, particularly domestic animals such as equine, bovine, ovine, feline, canine, murine, lagomorpha, and the like.

The formulation is administered at a dose effective to increase the response of T cells to antigenic stimulation. The response of activated T cells will be affected by the subject treatment to a greater extent than resting T cells. The determination of the T cell response will vary with the condition that is being treated. Useful measures of T cell activity are proliferation, the release of cytokines, e.g. IL-2, IFNg, TNFa; T cell expression of markers such as CD25 and CD69; and other measures of T cell activity as known in the art.

### Selection and Preparation of CTLA-4 Blocking Agents

CTLA-4 blocking agents are molecules that specifically bind to the extracellular domain of CTLA-4 protein, and block the binding of CTLA-4 to its counter-receptors, *e.g.* CD80, CD86, *etc.* Usually the binding affinity of the blocking agent will be at least about 100 µM. The blocking agent will be substantially unreactive with related molecules to CTLA-4, such as CD28 and other members of the immunoglobulin superfamily. Molecules such as CD80 and CD86 are therefore excluded as blocking agents. Further, blocking agents do not activate CTLA-4 signaling. Conveniently, this is achieved by the use of monovalent or bivalent binding molecules. It will be understood by one of skill in the art that the following discussions of cross-reactivity and competition between different molecules is intended to refer to molecules having the same species of origin, *e.g.* human CTLA-4 binds human CD80 and 86, *etc.*

Candidate blocking agents are screened for their ability to meet this criteria. Assays to determine affinity and specificity of binding are known in the art, including competitive and non-competitive assays. Assays of interest include ELISA, RIA, flow cytometry, *etc.* Binding assays may use purified or semipurified CTLA-4 protein, or alternatively may use T cells that express CTLA-4, *e.g*. cells transfected with an expression construct for CTLA-4; T cells that have been stimulated through cross-linking of CD3 and CD28; the addition of irradiated allogeneic cells, *etc.* As an example of a binding assay, purified CTLA-4 protein is bound to an insoluble support, *e.g.* microtiter plate, magnetic beads, *etc.* The candidate blocking agent and soluble, labeled CD80 or CD86 are added to the cells, and the unbound components are then washed off. The ability of the blocking agent to compete with CD80 and CD86 for CTLA-4 binding is determined by quantitation of bound, labeled CD80 or CD86. Confirmation that the blocking agent does not cross-react with CD28 may be performed with a similar assay, substituting CD28 for CTLA-4. Suitable molecules will have at least about 10' less binding to CD28 than to CTLA-4, more usually at least about 10⁴ less binding.

Generally, a soluble monovalent or bivalent binding molecule will not activate CTLA-4 signaling. A functional assay that detects T cell activation may be used for confirmation. For example, a population ofT cells may be stimulated with irradiated allogeneic cells expressing CD80 or CD86, in the presence or absence of the candidate blocking agent. An agent that blocks CTLA-4 signaling will cause an increase in the T cell activation, as measured by proliferation and cell cycle progression, release of IL-2, upregulation of CD25 and CD69, *etc.* It will be understood by one of skill in the art that expression on the surface of a cell, packaging in a liposome, adherence to a particle or well, etc. will increase the effective valency of a molecule.

Blocking agents are antibodies. Antibodies may be polyclonal or monoclonal; intact or truncated, *e.g.* F(ab'),, Fab, Fv; xenogeneic, allogeneic, syngeneic, or modified forms thereof, *e*.*g*. humanized, chimeric, *etc*.

The blocking agent is therefore an antibody or fragment thereof. Generally, the affinity will be at least about 10⁻⁶, more usually about 10⁻⁸ M, *i.e.* binding affinities normally observed with specific monoclonal antibodies.

A number of screening assays are available for blocking agents. The components of such assays will typically include CTLA-4 protein; and optionally a CTLA-4 activating agent, *e.g.* CD80, CD86, *etc.* The assay mixture will also comprise a candidate pharmacological agent. Generally a plurality of assay mixtures are run in parallel with different agent concentrations to obtain a differential response to the various concentrations. Typically, one of these concentrations serves as a negative control, i.e. at zero concentration or below the level of detection.

Conveniently, in these assays one or more of the molecules will be joined to a label, where the label can directly or indirectly provide a detectable signal. Various labels include radioisotopes, fluorescers, chemiluminescers, enzymes, specific binding molecules, particles, e.g. magnetic particles, and the like. Specific binding molecules include pairs, such as biotin and streptavidin, digoxin and antidigoxin etc. For the specific binding members, the complementary member would normally be labeled with a molecule which provides for detection, in accordance with known procedures.

One screening assay of interest is directed to agents that interfere with the activation of CTLA-4 by its counter-receptors. Quantitation of activation may be achieved by a number of methods known in the art. For example, the inhibition of T cell activation may be determined by quantitating cell proliferation, release of cytokines, etc.

Other assays of interest are directed to agents that block the binding of CTLA-4 to its counter-receptors. The assay mixture will comprise at least a portion of the natural counter-receptor, or an oligopeptide that shares sufficient sequence similarity to provide specific binding, and the candidate pharmacological agent. The oligopeptide may be of any length amenable to the assay conditions and requirements, usually at least about 8 aa in length, and up to the full-length protein or fusion thereof. The CTLA-4 may be bound to an insoluble substrate. The substrate may be made in a wide variety of materials and shapes e.g. microtiter plate, microbead, dipstick, resin particle, etc. The substrate is chosen to minimize background and maximize signal to noise ratio. Binding may be quantitated by a variety of methods known in the art. After an incubation period sufficient to allow the binding to reach equilibrium, the insoluble support is washed, and the remaining label quantitated. Agents that interfere with binding will decrease the detected label.

A variety of other reagents may be included in the screening assay. These include reagents like salts, neutral proteins, e.g. albumin, detergents, etc which may be used to facilitate optimal protein-DNA binding and/or reduce non-specific or background interactions. Also reagents that otherwise improve the efficiency of the assay, such as protease inhibitors, nuclease inhibitors, anti-microbial agents, etc. may be used.

Suitable antibodies for use as blocking agents are obtained by immunizing a host animal with peptides comprising all or a portion of CTLA-4 protein. Suitable host animals include mouse, rat, sheep, goat, hamster, rabbit, etc. The origin of the protein immunogen may be mouse, human, rat, monkey etc. The host animal will generally be a different species than the immunogen, e.g. mouse CTLA-4 used to immunize hamsters, human CTLA-4 to immunize mice, *etc.* The human and mouse CTLA-4 contain highly conserved stretches in the extracellular domain (Harper et al. (1991) J. Immunol. 147:1037-1044). Peptides derived from such highly conserved regions may be used as immunogens to generate cross-specific antibodies.

The immunogen may comprise the complete protein, or fragments and derivatives thereof. Preferred immunogens comprise all or a part of the extracellular domain of human CTLA-4 (amino acid residues 38-161), where these residues contain the post-translation modifications, such as glycosylation, found on the native CTLA-4. Immunogens comprising the extracellular domain are produced in a variety ofways known in the art, e.g. expression of cloned genes using conventional recombinant methods, isolation from T cells, sorted cell populations expressing high levels of CTLA-4, *etc*.

Where expression of a recombinant or modified protein is desired, a vector encoding the desired portion of CTLA-4 will be used. Generally, an expression vector will be designed so that the extracellular domain of the CTLA-4 molecule is on the surface of a transfected cell, or alternatively, the extracellular domain is secreted from the cell. When the extracellular domain is to be secreted, the coding sequence for the extracellular domain will be fused, in frame, with sequences that permit secretion, including a signal peptide. Signal peptides may be exogenous or native. A fusion protein of interest for immunization joins the CTLA-4 extracellular domain to the constant region of an immunoglobulin. For example, a fusion protein comprising the extracellular domain of mouse CTLA-4 joined to the hinge region of human Cg 1 (hinge-CH2-CH3) domain may be used to immunize hamsters.

When the CTLA-4 is to be expressed on the surface of the cell, the coding sequence for the extracellular domain will be fused, in frame, with sequences encoding a peptide that anchors the extracellular domain into the membrane and a signal sequence. Such anchor sequences include the native CTLA-4 transmembrane domain, or transmembrane domains from other cell surface proteins, *e.g*. CD4, CDB, sIg, *etc.* Mouse cells transfected with the human CTLA-4 gene may be used to immunize mice and generate antibodies specific for the human CTLA-4 protein.

Monoclonal antibodies are produced by conventional techniques. Generally, the spleen and/or lymph nodes of an immunized host animal provide a source of plasma cells. The plasma cells are immortalized by fusion with myeloma cells to produce hybridoma cells. Culture supernatant from individual hybridomas is screened using standard techniques to identify those producing antibodies with the desired specificity. Suitable animals for production of monoclonal antibodies to the human protein include mouse, rat, hamster, etc. To raise antibodies against the mouse protein, the animal will generally be a hamster, guinea pig, rabbit, etc. The antibody may be purified from the hybridoma cell supernatants or ascites fluid by conventional techniques, e.g. affinity chromatography using CTLA-4 bound to an insoluble support, protein A sepharose, etc.

The antibody may be produced as a single chain, instead of the normal multimeric structure. Single chain antibodies are described in Jost et al. (1994) J.B.C. 269:26267-73, and others. DNA sequences encoding the variable region of the heavy chain and the variable region of the light chain are ligated to a spacer encoding at least about 4 amino acids of small neutral amino acids, including glycine and/or serine. The protein encoded by this fusion allows assembly of a functional variable region that retains the specificity and affinity of the original antibody.

For *in vivo* use, particularly for injection into humans, it is desirable to decrease the antigenicity of the blocking agent. An immune response of a recipient against the blocking agent will potentially decrease the period of time that the therapy is effective. Methods of humanizing antibodies are known in the art. The humanized antibody may be the product of an animal having transgenic human immunoglobulin constant region genes (see for example International Patent Applications WO 90/10077 and WO 90/04036). Alternatively, the antibody of interest may be engineered by recombinant DNA techniques to substitute the CH 1, CH2, CH3, hinge domains, and/or the framework domain with the corresponding human sequence (see WO 92/02190).

The use of Ig cDNA for construction of chimeric immunoglobulin genes is known in the art (Liu et al. (1987) P.N.A.S. 84:3439 and (1987) J. Immunol. 139:3521). mRNA is isolated from a hybridoma or other cell producing the antibody and used to produce cDNA. The cDNA of interest may be amplified by the polymerase chain reaction using specific primers (U.S. Patent nos. 4,683,195 and 4,683,202). Alternatively, a library is made and screened to isolate the sequence of interest. The DNA sequence encoding the variable region of the antibody is then fused to human constant region sequences. The sequences of human constant regions genes may be found in Kabat et al. (1991) Sequences of Proteins of Immunological Interest, N.I.H. publication no. 91-3242. Human C region genes are readily available from known clones. The choice of isotype will be guided by the desired effector functions, such as complement fixation, or activity in antibody-dependent cellular cytotoxicity. Preferred isotypes are IgG 1, IgG3 and IgG4. Either of the human light chain constant regions, kappa or lambda, may be used. The chimeric, humanized antibody is then expressed by conventional methods.

Antibody fragments, such as Fv, F(ab')₂ and Fab may be prepared by cleavage of the intact protein, e.g. by protease or chemical cleavage. Alternatively, a truncated gene is designed. For example, a chimeric gene encoding a portion of the F(ab'), fragment would include DNA sequences encoding the CH1 domain and hinge region of the H chain, followed by a translational stop codon to yield the truncated molecule.

Consensus sequences of H and L J regions may be used to design oligonucleotides for use as primers to introduce useful restriction sites into the J region for subsequent linkage of V region segments to human C region segments. C region cDNA can be modified by site directed mutagenesis to place a restriction site at the analogous position in the human sequence.

Expression vectors include plasmids, retroviruses, YACs, EBV derived episomes, and the like. A convenient vector is one that encodes a functionally complete human CH or CL immunoglobulin sequence, with appropriate restriction sites engineered so that any VH or VL sequence can be easily inserted and expressed. In such vectors, splicing usually occurs between the splice donor site in the inserted J region and the splice acceptor site preceding the human C region, and also at the splice regions that occur within the human CH exons. Polyadenylation and transcription termination occur at native chromosomal sites downstream of the coding regions. The resulting chimeric antibody may be joined to any strong promoter, including retroviral LTRs, *e.g.* SV-40 early promoter, *(*Okayama et al. (1983) Mol. Cell. Bio. 3:280), Rous sarcoma virus LTR(Gorman et al. (1982) P.N.A.S. 79:6777), and moloney murine leukemia virus LTR (Grosschedl et al. (1985) Cell 41:885); native Ig promoters, *etc.*

### Immune Response Stimulating Agents

The CTLA-4 blocking agent can be used alone or in combination with an immune response stimulating agent. As used herein, an "immune response stimulating agent" refers to any agent which directly or indirectly stimulates an immune response in combination with a CTLA-4 blocking agent. For example, immune response stimulating agents include cytokines as well as various antigens including tumor-specific antigens and antigens derived from pathogens. In addition, immune response stimulating agents include cytokine transduced tumor cells, e.g. tumor cells transduced with GM-CSF, as well as tumor cells which have been irradiated and/or treated with a chemotherapeutic agent *ex vivo* or *in vivo.* As demonstrated by the examples provided herein, immune response stimulating agents can have a significant effect on tumor treatment when used in combination with a CTLA-4 blocking agent.

In some instances cellular debris from dead or dying tumor cells provides immune response stimulation which can be combined *in vivo* or *ex vivo* with a CTLA-4 blocking agent. The use of chemotherapeutic agents is an example of production of an immune response stimulating agent by indirect means. Use of a source to irradiate tumor cells *ex vivo* or *in vivo* also constitutes a method which indirectly produces immune response stimulating agents, as does surgical reduction or cytoreduction, androgen ablatement (in prostate cancer) and estrogen ablatement (in breast cancer).

The basis for use of chemotherapeutic agents and the like with CTLA-4 blocking agents is as follows. As indicated in the examples, the CTLA-4 blockade works well with established tumors and increases the immunogenicity of irradiated tumor cells. This suggests that the CTLA-4 blockade can be combined with more conventional methods of cancer treatment to produce a synergistic effect. For example, the CTLA-4 blockade may be initiated shortly after treatment with a chemotherapeutic agent. The dose of the chemotherapeutic agent is adjusted to a level that kills a reasonable amount of the tumor mass and generates debris which act as an agent to stimulate an immune response by T cells as a result of CTLA-4 blockade. This allows the chemotherapeutic agent to be given at levels well below those now used to obtain maximal killing of the tumor cells, since the immune response facilitated by CTLA-4 eliminates the residual tumor mass. This minimizes the often gruesome side effects, including immunosuppression, associated with the conventional application of chemotherapy. Similar considerations apply to radiotherapy and ablation therapies. In prostate cancer, for example, androgen ablatement may be followed by surgical reduction and CTLA-4 blockade. The dose ofchemotherapeutic agent or radiation if used in conjunction with a CTLA-4 blocking agent is preferably less than half, more preferably between 2-20%, and still more preferably between 5-10% of the dose usually used.

When the CTLA-4 blocking agent is other than an antibody to the extracellular domain of CTLA-4 or a fragment thereof, *e.g.* Fab' fragment, such blocking agents can be used independently, i.e., without an immune response stimulating agent. However, CTLA-4 blocking agents, especially those which consist of an antibody to the extracellular portion of the CTLA-4, are preferably used in combination with one or more immune response stimulating agents. CTLA-4 blocking agents may also be used in conjunction with radiation and/or chemotherapeutic treatment which indirectly produces immune response stimulating agents. Such combined use can involve the simultaneous or sequential use of CTLA-4 blocking agent and immune response stimulating agent and can occur at different sites. For example, the CTLA-4 blocking agent can be administered at a site away from a tumor after the tumor has been directly irradiated. Alternatively, a chemotherapeutic agent can be used to treat tumor cells either locally or systemically followed by use of a CTLA-4 blocking agent.

The subject treatment may be performed in combination with administration of cytokines that stimulate antigen presenting cells, e.g. granulocyte/macrophage colony-stimulating factor (GM-CSF), macrophage colony-stimulating factor (M-CSF), granulocyte colony-stimulating factor (G-CSF), interleukin 3 (IL-3), interleukin 12 (IL-12), *etc.* Additional proteins and/or cytokines known to enhance T cell proliferation and secretion, such as IL-1, IL-2, B7, anti-CD3 and anti-CD28 can be employed simultaneously or sequentially with the blocking agents to augment the immune response.

### Tumor Antigens

Tumor cells whose growth may be decreased by administration of the subject blocking agents include carcinomas e.g. adenocarcinomas, which may have a primary tumor site in the breast, ovary, endometrium, cervix, colon, lung, pancreas, esophagus, prostate, small bowel, rectum, uterus or stomach; and squamous cell carcinomas, which may have a primary site in the lungs, oral cavity, tongue, larynx, eosophagus, skin, bladder, cervix, eyelid, conjunctiva, vagina, *etc.* Other classes of tumors that may be treated include sarcomas, e.g. myogenic sarcomas; neuromas; melanomas; leukemias, certain lymphomas, trophoblastic and germ cell tumors; neuroendocrine and neuroectodermal tumors.

Tumors of interest include those that present tumor-specific antigens. Such antigens may be present in an abnormal context, be uniquely expressed by the tumor cells, or may be a mutated form of a normal, unaltered self antigen. The tumor-specific antigen may be administered with the subject blocking agents to increase the host T cell response against the tumor cells. Such antigen preparations may comprise purified protein, or lysates from tumor cells.

Examples oftumor antigens are cytokeratins, particularly cytokeratin 8,18 and 19, as an antigen for carcinomas. Epithelial membrane antigen (EMA), human embryonic antigen (HEA-125); human milk fat globules, MBr1, MBr8, Ber-EP4,17-1A, C26 and T16 are also known carcinoma antigens. Desmin and muscle-specific actin are antigens of myogenic sarcomas. Placental alkaline phosphatase, beta-human chorionic gonadotropin, and alpha-fetoprotein are antigens of trophoblastic and germ cell tumors. Prostate specific antigen is an antigen of prostatic carcinomas, carcinoembryonic antigen of colon adenocarcinomas. HMB-45 is an antigen of melanomas. In cervical cancer, useful antigens could be encoded by human papilloma virus. Chromagranin-A and synaptophysin are antigens of neuroendocrine and neuroectodermal tumors. Of particular interest are aggressive tumors that form solid tumor masses having necrotic areas. The lysis of such necrotic cells is a rich source of antigens for antigen-presenting cells.

The subject therapy may be combined with the transfection of tumor cells or tumor-infiltrating lymphocytes with genes encoding for various cytokines or cell surface receptors (see Ogasawara et al. (1993) Cancer Res. 53:3561-8; and Townsend et al. (1993) Science 259:368-370). For example, it has been shown that transfection of tumor cells with cDNA encoding CD80 leads to rejection of transfected tumor cells, and can induce immunity to a subsequent challenge by the non-transfected parent tumor cells (Townsend et al. (1994) Cancer Res. 54:6477-6483). The subject therapy enhances this effect.

Tumor-specific host T cells may be combined *ex vivo* with the subject blocking agents, and tumor antigens or cells and reinfused into the patient. When administered to a host, the stimulated cells induce a tumoricidal reaction resulting in tumor regression. The host cells may be isolated from a variety of sources, such as lymph nodes, *e.g*. inguinal, mesenteric, superficial distal auxiliary, *etc.;* bone marrow; spleen; or peripheral blood, as well as from the tumor, *e.g*. tumor infiltrating lymphocytes. The cells may be allogeneic or, preferably, autologous. For *ex vivo* stimulation, the host cells are aseptically removed, and are suspended in any suitable media, as known in the ait. The cells are stimulated by any of a variety of protocols, particularly combinations of B7, anti-CD28, *etc.,* in combination with the blocking agents. The stimulated cells are reintroduced to the host by injection, e.g. intravenous, intraperitoneal, etc. in a variety of pharmaceutical formulations, including such additives as binder, fillers, carriers, preservatives, stabilizing agents, emulsifiers and buffers. Suitable diluents and excipients are water, saline, glucose and the like.

### Self Antigens

Of particular interest are poorly immunogenic and non-immunogenic tumors, which present special challenges due to their failure to provide adequate antigenic stimulation to provoke an immune response. In these cases, a self antigen common to both normal and cancerous tissue can be targeted, or alternatively normal gene products with highly restricted cellular distribution. In this aspect of the invention, a self antigen preparation is administered in combination with the CTLA-4 blocking agents to help stimulate an autoreactive peripheral T cell response against the cells expressing the self antigen or gene product.

The self antigen preparation may comprise a mixture of antigens, such as tumor lysates or irradiated tumor cell vaccines, as well as purified antigen comprising a specific self antigen of interest (e.g., protein, carbohydrate and the like) or a mixture of self antigens. A "purified" antigen comprising a protein is distinguished from naturally occurring protein by at least one or more characteristics. For example, the protein may be isolated or purified away from some or all of the proteins and compounds with which it is normally associated in its wild type host, and thus may be substantially pure. For example, a purified protein is unaccompanied by at least some of the material with which it is normally associated in its natural state, preferably constituting at least about 0.5%, more preferably at least about 5% by weight of the total protein in a given sample. A substantially pure protein comprises at least about 75% by weight of the total protein, with at least about 80% being preferred, and at least about 90% being particularly preferred.

Preferably, the self antigen is a tissue-specific self antigen. Self antigens that may find use in the subject therapy include tyrosinase, trp1, trp2, melanA/MART 1, gp 100 and other proteins involved in melanin synthesis; prostate specific antigen (PSA), prostatic acid phosphatase (PAP), prostate specific membrane antigen (PMSA), prostate stem cell antigen (PSCA), prostase or other prostate-specific gene products; Her2/neu or other mammary-specific gene products. Additional self antigens that serve as targets for the immune responses elicited by the subject therapy can be identified by methods well known in the art, such as expression cloning, to allow immunization against defined antigens of known distribution and provide a more focused immune response. Alternatively, the antigen preparation may comprise modified self antigen or antigens or a xenogeneic antigen (*e.g*., the corresponding murine version of a human antigen of interest) to assist in breaking immune tolerance to the self antigen(s).

The immune response stimulating agents outlined above may also be incorporated into the subject therapy to augment the response to self antigen. In a preferred embodiment, for example, the antigen preparation comprises tumor vaccines comprising irradiated tumor cells transduced with genes of interest, such as genes encoding for cytokines that stimulate antigen presenting cells, e.g. granulocyte-macrophage colony stimulating factor (GM-CSF), macrophage colony stimulating factor (M-CSF), or granulocyte colony stimulating factor (G-CSF). More conventional therapies such as chemotherapy, radiotherapy or androgen ablation may also be employed to induce sufficient tumor cell death to achieve some priming of tumor-reactive T cells, as discussed above. Similarly, alternative costimulation strategies such as dendritic cells pulsed with peptides or RNA to provide immunization in the context of a potent APC, or anti-CD40 antibodies to enhance expression of co-stlmulatory ligands on APCs, may also be employed.

The treatment provides a method for breaking immune tolerance and mounting an effective and controlled cytotoxic response against a desired self antigen or antigens. This immunological therapy will find particular use with tumors such as melanoma, mammary cancer, testicular cancer, ovarian cancer, prostate cancer and the like where loss or modification of some or all of the normal tissue is an acceptable, or even a desirable, side effect. Alternatively, the treatment may be used to enhance or effect antigen ablatement of a selected tissue, as a prophylactic measure against cancer development or for other medical reasons. An immunological method of selective tissue ablatement offers considerable advantages over more invasive surgical methods.

Administration of the subject blocking agents may be contra-indicated for certain lymphomas. In particular, T cell lymphomas may not benefit from increased activation. CD80 antigen is strongly expressed by the Reed-Sternberg cells in Hodgkin's disease, which are frequently surrounded by CD28-expressing T cells (Delabie et al. (1993) Blood 82:2845-52). It has been suggested that the accessory cell function of Reed-Sterrxberg cells leads to T cell activation, and contributes to the Hodgkin's syndrome.

Many conventional cancer therapies, such as chemotherapy and radiation therapy, severely reduce lymphocyte populations. While the subject therapy may alleviate this immunosuppression to some extent, a preferred course of combined treatment will use such lymphotoxic therapies before or after the subject therapy.

### Adjuvants

Adjuvants potentiate the immune response to an antigen. The CTLA-4 blocking agents are used as an adjuvant to increase the activation of T cells, and to increase the class switching of antibody producing cells, thereby increasing the concentration of IgG class antibodies produced in response to the immunogen. The blocking agents are combined with an immunogen in a physiologically acceptable medium, in accordance with conventional techniques for employing adjuvants. The immunogen may be combined in a single formulation with the blocking agent, or may be administered separately. Immunogens include polysaccharides, proteins, protein fragments, haptens, *etc*. Of particular interest is the use with peptide immunogens. Peptide immunogens may include tumor antigens and viral antigens or fragments thereof, as described above.

The use of the subject blocking agents in conjunction with genetic immunization is also of interest. A DNA expression vector encoding a peptide or protein antigen of interest is injected into the host animal, generally in the muscle or skin. The gene products are correctly glycosylated, folded and expressed by the host cell. The method is advantageous where the antigens are difficult to obtain in the desired purity, amount or correctly glycosylated form or when only the genetic sequences are known. Typically, DNA is injected into muscles or delivered coated onto gold microparticles into the skin by a particle bombardment device, a "gene gun". Genetic immunization has demonstrated induction of both a specific humoral but also a more broadly reacting cellular immune response in animal models of cancer, mycoplasma, TB, malaria, and many virus infections including influenza and HIV. See, for example, Mor et al. (1995) J Immunol 155:2039-46; Xu and Liew (1995) Immunology 84:173-6; and Davis et al, (1994) Vaccine 12:1503-9.

### Administration and Formulation

Various methods for administration may be employed. The CTLA-4 blocking agent formulation may be injected intravascularly, subcutaneously, peritoneally, etc. The dosage of the therapeutic formulation will vary widely, depending upon the nature of the disease, the frequency of administration, the manner of administration, the purpose of the administration, the clearance of the agent from the host, and the like. The dosage administered will vary depending on known factors, such as the pharmacodynamic characteristics of the particular agent, mode and route of administration, age, health and weight of the recipient, nature and extent of symptoms, concurrent treatments, frequency of treatment and effect desired. The dose may be administered as infrequently as weekly or biweekly, or fractionated into smaller doses and administered daily, semi-weekly, *etc.* to maintain an effective dosage level. Generally, a daily dosage of active ingredient can be about 0.1 to 100 mg/kg of body weight. Dosage forms suitable for internal administration generally contain from about 0.1 mg to 500 mgs of active ingredient per unit. The active ingredient may vary from 0.5 to 95% by weight based on the total weight of the composition.

In some cases it may be desirable to limit the period of treatment due to excessive T cell proliferation. The limitations will be empirically determined, depending on the response of the patient to therapy, the number of T cells in the patient, the type of antigen targeted by the subject therapy, etc. The number ofT cells may be monitored in a patient by methods known in the art, including staining with T cell specific antibodies and flow cytometry.

The CTLA-4 blockers are prepared as formulations at an effective dose in pharmaceutically acceptable media, for example normal saline, vegetable oils, mineral oil, PBS, etc. Therapeutic preparations may include physiologically tolerable liquids, gel or solid carriers, diluents, adjuvants and excipients. Additives may include bactericidal agents, additives that maintain isotonicity, e.g. NaCl, mannitol; and chemical stability, e.g. buffers and preservatives. or the like. The CTLA-4 blockers may be administered as a cocktail, or as a single agent. For parenteral administration, the blocking agent may be formulated as a solution, suspension, emulsion or lyophilized powder in association with a pharmaceutically acceptable parenteral vehicle. Liposomes or non-aqueous vehicles, such as fixed oils, may also be used. The formulation is sterilized by techniques as known in the art.

The functional effect of CTLA-4 blockade may also be induced by the administration of other agents that mimic the change in intra-cellular signaling observed with the subject invention. For example, it is known that specific cytoplasmic kinases may be activated in response to binding of extracellular receptors. Agents that block the kinase activity would have a similar physiological effect as blocking receptor binding. Similarly, agents that increase cyclic AMP, GTP concentrations and intracellular calcium levels can produce physiological effects that are analagous to those observed with extracellular receptor binding.

The following examples are offered by way of illustration and not by way of limitation. Only Example 10 contains an example of treatment of established metastases.

### Experimental

### EXAMPLE 1

### Generation of Monoclonal Antibodies Reactive With Mouse CTLA-4

### a) Preparation of a Mouse CTLA-4 Immunogen

A fusion protein comprising the extracellular portions of the mouse CTLA-4 gene and the constant region of human IgGI, termed mCTLA4-Hgl, was obtained from Drs. P. Lane and K. Karjalainen (Basel Institute for Immunology, Basel, Switzerland). An expression vector capable of expressing the mCTLA4-Hg 1 protein was constructed as described [Lane, et al. Immunol. 80:56 (1993)]. Briefly, sequences encoding the extracellular portions of the mouse CTLA-4 molecule were generated using PCR. The following primer pair was used to amplify these CTLA-4 sequences from a plasmid containing mouse CTLA-4 sequences: 5'-TTACTCTACTCCCTGAGG AGCTCAGCACATTTGCC-3'(SEQ ID NO:1) and 5'-TATACTTACCAGAATCCG GGCATGGTTCTGGATCA-3' (SEQ ID NO:2). The amplified CTLA-4 sequences were then inserted into an expression vector that permits the insertion of a gene of interest upstream of sequences encoding the hinge, CH2 and CH3 domains of the human IgG 1 protein [Traunecker, et al. Trends Biotech. 9:109 (1991)]. Each primer contained appropriate restriction sites for subcloning into the human IgG1 expression vector, together with a 3' splice donor site within the 3' primer to splice to the human gl exons correctly. The plasmid containing sequences encoding the mCTLA-4-Hgl fusion protein was termed pH β-APr-1-neo-mCTLA4-Hgl. The amino acid sequence of the mCTLA4- Hgl protein is listed in SEQ ID NO:3.

To express the mCTLA4-Hgl protein, the pHβAPr-1-neo-mCTLA4- Hgl expression vector was transfected into the mouse plasmacytoma line, J558L (J558L is identical to the J558 cell line which is available from ATCC [ATCC TIB 6]) using the standard technique of protoplast fusion. J558L cells were cultured at 5 x 10⁴ cells/ml. Transfected J558L cells were then selected in the presence of medium containing xanthine (Sigma) and mycophenolic acid (Calbiochem, LaJolla, CA) (selective medium). The selective medium was applied 24 hr after transfection and positive clones (ie., clones which grew in the selective medium) were screened two weeks later. Clones that secreted the fusion protein were identified using an ELISA for human IgG1. A good secreting clone was identified and designated clone no. 15. Clone no. 15 cells were metabolically labelled with [³⁵S]methionine and the secreted proteins were immunoprecipitated with protein A and the precipitated proteins were resolved on an SDS polyacrylamide gel. The mCTLA4-Hgl protein was found to migrate on SDS-PAGE gels as a monomer of approximately 60,000 MW under reducing conditions and as a dimer under non-reducing conditions.

Purified preparations of mCTLA4-Hg 1 protein were obtained by affinity chromatography of culture supernatants of clone no. 15 cells on a protein A-Sepharose (Zymed, South San Francisco, CA) column. Briefly, J558 cells expressing the mCTLA4-Hgl protein were grown in IMDM supplemented with 5% FCS, glutamine, 2ME and antibiotics. Culture supernatants were collected from the cells and centrifuged at 1500 x g to remove any remaining cells and the clarified supernatant was filtered through a 0.4 micron pore size. The filtered supernatant was adjusted to pH 8.5 using 1N NaOH; the supernatant was then passed over a 2 ml (packed volume) protein A-Sepharose column at a flow rate of 2 ml/min. It is noted that the J558 cell line produces an additional immunoglobulin (i.e., besides the mouse CTLAIg fusion protein) that binds to protein G; therefore the use of protein G resins is not recommended for the purification of the mCTLA4-Hgl protein from transfected J558 cells.

The protein A column was washed with 20 to 30 column volumes of PBS and the fusion protein was eluted with 50 mM diethylamine (pH 11.0). Two milliliter fractions were collected into tubes containing 0.2 ml 1M Tris-HCl to neutralize the pH of the sample. The absorbance at 280 nm was determined and used to assess the protein concentration of each fraction. Fractions containing protein were combined and dialyzed overnight against 2 to 3 changes of PBS (1 liter per change). The presence of mCTLA4-Hgl protein was confirmed by SDS-PAGE, which showed a band of approximately 40 kD (the predicted molecular weight of the fusion protein). In addition, the purified mCTLA4-Hgl protein was tested in an ELISA using an antihuman IgG1 antibody (HP6058; the HP6058 hybridoma (ATCC CRL 1786) was used as the source of HP6058 antibodies).

### b) Immunization of Hamsters

To immunize hamsters with the mouse CTLA-4 fusion protein, purified mCTLA4-Hgl protein (hereafter referred to as CTLA-4Ig) was used to coat heat-killed *Staphylococcus aureus* (StaphA) bacteria cells (Calbiochem, LaJolla, CA). Six week old Golden Syrian hamsters (Harlan Sprague Dawley, Indianapolis, IN) were injected in the footpad with 50µl (packed volume) of heat-killed StaphA bacteria coated with approximately 100 µg of CTLA-4Ig suspended in 0.2 ml of PBS. The StaphA cells were coated as follows.

StaphA cells were prepared according to the manufacturer's protocol to a concentration of 10% w/v in saline (0.9% NaCI). One ml of the bacterial cell slurry was centrifuged at 1,400 x g to pellet the bacteria and the supernatant was removed. A 1 ml solution containing approximately 100 µg of purified CTLA-4Ig in PBS was added to the pellet and the mixture was incubated at 37°C for 2 hours with agitation. The bacteria were then pelleted by centrifugation as described above; the pellet was washed twice with 1 ml of PBS/wash. The CTLA-4Ig-coated bacterial cells were then resuspended in approximately 200 µl of PBS; 50 µl of this preparation was injected per footpad.

A total of five injections were given per hamster. On the day of the final boost and prior to the injection, approximately 100 µl of serum was obtained by intraocular bleeding performed by the Office of Laboratory Animal Care staff (Univ. of Calif, Berkeley). This serum was analyzed in comparison to serum obtained by the identical methodology prior to the first injection.

A CTLA-4Ig binding ELISA was utilized to demonstrate the presence of antibody that recognized the CTLA-4Ig fusion protein in the post-immunization bleed. The CTLA-4Ig binding ELISA was conducted as follows. CTLA-4Ig fusion protein or CD4Ig fusion protein was used to coat the wells of 96 well modified flatbottom ELISA plates (Coming, Coming, NY).

CD4Ig is a fusion protein that consists of the extracellular domain of mouse CD4 and the hinge, CH2 and CH3 domains of human IgG1 [Traunecker et al., supra.]; the CD4Ig protein was used as a negative control in the ELISA assays. The CD4Ig fusion protein was prepared from transfected J558 cells and purified by affinity chromatography on protein A Sepharose as described for the mCTLA4-Hµl (i.e., the CTLA-4Ig) fusion protein in section (a) above.

Fifty microliters of the fusion proteins, at a concentration of 1 µg/ml in 0.4% gelatin in PBS were placed in the wells. The plates were incubated at 37°C for 2-3 hours to allow the proteins to absorb; the plates were then washed three times using 150 µl of 0.9% NaCl containing 0.05% Tween-20. The remaining protein binding sites in the wells were then blocked using 0.4% gelatin in PBS (blocking buffer) for 30 min at 37°C; following the blocking step, the plates were washed twice with 0.9% NaCl containing 0.05% Tween-20. Fifty microliters of solution containing antiCTLA-4 antibodies (i.e., serum from immunized hamsters, purified antibodies or culture supernatants) were added into triplicate wells and the plates were incubated for 2-3 hours at 37°C. To assess the amount of anti-CTLA-4 antibodies present in the serum of immunized hamsters, the initial post-immunization bleeds were tested using dilutions ranging from 1:1000 to 1:100 (diluted into PBS containing 0.4% gelatin).

The wells were then washed three times using 150 µl of 0.9% NaCl containing 0.05% Tween-20. Fifty microliters of a solution containing goat anti-hamster IgG polyclonal sera conjugated to horseradish peroxidase (CalTag, South San Francisco, CA) at a concentration of 1 µg/ml in blocking buffer was added to the wells and the plates were incubated for 1 hour at 37°C. The plates were then washed four times with 0.9% NaCl containing 0.05% Tween-20. A solution containing 0.55 mg/ml ABTS 2,2'-Azino-bis (3-ethylbenzthiazoline-6-sulfonic acid)] in citrate buffer [0.1 M citric acid (pH 4.35)] was added and the plates were incubated for approximately 20 min at 37°C. The plates were then read at 405 nm using a BioTech plate reader (Beckman Instruments, Palo Alto, CA) to assess the absorbance of the green reaction product.

The results of the CTLA-4Ig binding ELISA demonstrated the presence of antibody that recognized the CTLA-4Ig fusion protein in the post-immunization bleed at serum dilutions 1000-fold greater than the dilution at which background could be detected using the pre-immune bleed.

### c) Isolation of Hybridoma Lines Secreting Anti-mouse CTLA-4 Antibodies

Three days following the final injection, draining lymph nodes were removed from the hamsters. Lymphocytes were isolated from the popliteal lymph nodes which drain the hind-limbs. Cell suspensions were made from the isolated lymph nodes as follows. The dissected nodes were placed in a tissue culture dish (Falcon Plastics, Mountain View, CA) containing RPMI medium (GibcoBRL, Gaithersburg, MD) supplemented with 10% FCS (BioWhittaker, Walkersville, MD). Lymphocytes were released from the nodes by gentle grinding of the nodes with frosted glass slides; the lymphocyte suspensions were counted using a hemocytometer.

The lymphocytes isolated from the immunized hamsters were fused to the fusion cell partner, P3X3.Ag8.653 (ATCC CRL 1580). P3X3.Ag8.653 cells were split 1:20 every 3 days prior to the fusion in IMDM (Univ. of Calif., San Francisco Tissue Culture Facility) containing 20% FCS (fetal calf serum) (BioWhittaker, Walkersville, MD), 50 µM 2-ME, 50 µM gentamicin.

The fusion with the myeloma line used a standard polyethylene glycol fusion technique [McKeam et al., Immunol. Rev. 47:91 (1979)]. Briefly, sterile lymphocyte cell suspensions were prepared in serum free Iscove's Modified Dulbecco's Media (IMDM). The lymphocytes were washed twice with IMDM and adjusted to a density of 12.5 x 10⁶ cells/ml.

P3X3.Ag8.653 cells (grown as described above) were washed twice with serum free IMDM [these cells were centrifuged for 5 minutes at 1000 r.p.m. in a TJ-6 centrifuge (Beckman Instruments, Palo Alto, CA) at 25°C to pellet the cells] and the P3X3.Ag8.653 cell density was adjusted to 5 x 10⁶ cells/ml.

Four milliliters of the lymphocyte cell suspension were mixed with 1 ml of the washed P3X3.Ag8.653 cells in 60 mm tissue culture dish (Falcon). The tissue culture dishes were placed in microtiter plate carriers (Beckman Instruments, Palo Alto, CA) and centrifuged at 250 x g (1200 r.p.m.; TJ-6 centrifuge) for 5 minutes to generate an adherent monolayer of cells on the bottom of the dish. The supernatant was aspirated from the dishes and the dishes were neatly flooded with I ml of 50% polyethylene glycol (PEG 1500, Boehringer Mannheim) in IMDM; the PEG solution was prepared by warming 4 ml of PEG 1500 and 4 ml of IMDM separately in 60°C water bath and then combining by aspiration of the PEG into a pipette followed by the IMDM and mixing thoroughly. After 30 seconds at room temperature, the dishes were flooded with 5 ml of serum free IMDM.

Following the final wash on the day of the fusion, the cells were left in the 60 mm dish with 5 ml of IMDM medium containing FCS for 12 hours at 37°C with 5% CO₂. On the following day, the fused cells were diluted into 100 ml of IMDM containing 20% FCS and 1 X HAT media (Boehringer Mannheim, NJ) and 100 µl was plated per well in 96 well flat bottom plates. After 5 and 9 days, an additional 50 µl of media was added to each well. Thereafter, 50 µl of media was removed and fresh media added at 3 day intervals. Once cell numbers were within the 1000-5000 per well range, hybridoma supernatants were tested for reactivity to CTLA-4Ig and for a lack of reactivity to CD4Ig by ELISA as described in section (b) above. Hybridoma supernatants were used undiluted in the ELISA (50 µl/well).

Hybridomas from positive wells were repetitively cloned by limiting dilution in the presence of irradiated mouse thymocyte feeder layers. A hybridoma line secreting a monoclonal antibody, termed antibody 9H 10, was selected by the following criteria: 1) reactivity against CTLA-4Ig but not CD4Ig in ELISAs; 2) the ability to block CTLA-4Ig binding to B7 transfectants; 3) the ability to stain activated T cells but not freshly isolated T cells; and 4) the ability to stain a CTLA-4 transfectant but not control transfectants.

The ability of antibody 9H10 to block CTLA4Ig binding to B7 transfectants was demonstrated as follows. Approximately 10 µl of mAb 9H 10 was incubated at 22°C for 30 min with 1 µg of CTLA-4Ig fusion protein in a final volume of 50 µl of a solution comprising PBS. To this mixture was added 2 x 10⁵ B7-EL-4 cells, suspended in 10 µl ice-cold PBS containing 1% calf serum and 0.05% sodium azide. B7-EL-4 cells are the C57BL/6-derived EL4 thymoma cell line transfected with an expression vector encoding the mouse B7 cell surface protein, as described in Townsend et al. Cancer Res. 54:6477-83 (1994).

The resulting mixture was then incubated on ice for 30 minutes, followed by two washes with 4 ml/wash of PBS containing 1% calf serum and 0.05% sodium azide. The cells were then stained with fluorescein isothiocynate (FITC)-conjugated anti-human IgG (Caltag, South San Francisco, CA). As a negative control for this experiment, the CTLA-41g fusion protein was incubated with either a control hamster IgG or the EL-4 parent cell line. The cells were analyzed on a FACScan (BectonDickinson, Mountain View, CA); the LYSIS II program (Becton Dickinson) was used to electronically gate on relevant populations. In most experiments, 10,000 live gated events were collected for analysis. The results showed that the 9H 10 antibody blocked CTLA-4 binding to B7-EL-4 cells.

The ability of the 9H10 antibody to stain activated T cells but not freshly isolated T cells was demonstrated as follows. Fresh and activated splenocytes were generated. Spleens from 4-6 week BALB/c mice were harvested and minced, and suspensions were treated with hemolytic Gey's solution to remove the red blood cells, a standard technique in the art [Mishell and Shiigi, Selected Methods in Cellular Immunology, W.H. Freeman and Co., San Francisco (1980) pp.23-24]. The cells were cultured in RPMI containing 10% fetal calf serum, with soluble anti-CD-3 antibody at 10 µg/ml added to activate one portion of the cell population. The other portion of the splenocytes was not treated with anti-CD3 and represents fresh (but not activated splenocytes). The two cell populations were then stained with either 1) a combination of FITC-conjugated 9H10 (the anti-CTLA-4 antibody; 5 µg of antibody) and PE-conjugated Thyl.2 or 2) a combination of FITC-conjugated hamster Ig and PE-conjugated Thyl.2. The data were analyzed on a FACScan and was electronically gated for Thyl.2 positive cells to analyze only the relevant T cell population. The results of this experiment demonstrated that the 9H10 antibody stained activated (i.e., CTLA-4 expressing) but not freshly isolated T cells.

The ability of the 9H10 antibody to stain a CTLA-4 transfectant but not control transfectants was demonstrated as follows. A parent CHO (Chinese Hamster Ovary, CHO-K1 cells) cell line (ATCC CCL 61) was transfected with pSRlneo.CTLA-4. pSRlneo.CTLA-4 contains the entire 1.9 kb cDNA encoding the mouse CTLA-4 protein [Brunet et al., *Nature* 328:267 (1987)] inserted into the pSRlneo expression vector. Cells transfected with the pSRlneo.CTLA vector express the mouse CTLA-4 protein on the cell surface.

The parent (i.e., CHO-K1 cells) and transfected cells were stained either 1) a combination of FITC-conjugated 9H 10 (the anti-CTLA-4 antibody; 5 µg of antibody) and PE-conjugated Thyl.2 or 2) a combination of FITC-conjugated hamster Ig and PE-conjugated Thy1.2. The data was electronically gated for Thy1.2 positive cells to analyze only the relevant T cell population. The results of this experiment demonstrated that the 9H 10 antibody stains CTLA-4 transfectants but not control transfectants.

The above results demonstrated that the 9H 10 monoclonal antibody reacts specifically with the mouse CTLA-4 protein.

### EXAMPLE 2

### Anti-CTLA-4 Monoclonal Antibodies Cause Rejection of V51BLim 10 Tumors in Mice

The anti-mouse CTLA-4 monoclonal antibody, 9H10, was used to treat mice that received injections of a colon carcinoma cell line. The injection of the 9H10 mAb along with V51BLim 10 tumor cells resulted in the complete rejection of the tumor cells in the experimental animals. In contrast, mice injected with an anti-CD28 mAb and V51BLim 10 cells or mice injected with V51BLim10 cells alone both developed tumors which exhibited a steady increase in average tumor size over a period of four weeks.

### a) Generation of the v51BLim10 Cell Line

The V51BLim10 cell line was generated by transfection of the SRlneo expression vector into the 5 1BLim10 cell line. The 5 1BLim cell line is a colon carcinoma cell line that provides an accurate animal model for colon cancer metastasis in humans. Bresalier, et al., Cancer Res. 47:1398 (1987).

The V51BLim10 cell line used in the present experiments was generated as follows. The murine colon cancer cell line 51B established by Corbett et al., Cancer Res. 35:2434-9 (1975) was injected into the cecal wall of BALB/c mice; the resulting colonic tumors were found to spontaneously metastasize to the liver in a minority of the injected mice. Bresalier et al., Cancer Res. 47:1398 (1987). Tumor cell lines having progressively increased metastatic activity were developed by collecting cells from the original metastases, which were then used for successive reinjection into the ceca of additional mice. These cell lines were termed 51BLim-1 through 5 1BLim-5 where the number following the dash refers to the number of metastatic cycles.

A 51B metastatic derivative obtained from Dr. Warren at the University of California San Francisco was designated 51BLim 10; the 51 BLim 10 cell line corresponds to the 51BLiM5 cell line described by Bresalier, et al., Cancer Res. 47:1398 (1987).

The SR1neo expression vector was transfected into the 51 BLiM-10 cell line to generate the V51BLim10 cell as described [Townsend et al. Cancer Res. 54:6477-83 (1994)]. The SR1neo expression vector (obtained from L. Lanier at DNAX Research Institute of Molecular and Cellular Biology, Palo Alto, CA) allows the expression of a gene of interest under the transcriptional control of the HTLV-1 LTR. The SRlneo vector also contains the neo gene under the transcriptional control of the SV40 promoter/enhancer. The presence of the neo gene allows for the selection of transfected cells containing the SRlneo vector.

The SRlneo expression vector was transfected into 51 BLiM-10 cells by electroporation using a BTX T 800 electroporator (BTX, Inc., San Diego, CA). Five pulses for 99 µs each at 450 or 600 V were applied. The electroporation was carried out in a final reaction volume of 750 µl of a solution comprising 270 mM sucrose, 7mM NaPO₄(pH 7.4), 1 mM MgCl₂, 5 x 10⁶ 51B LiM-10 cells and 50 µg of the SRlneo expression vector. Following electroporation, the cells were cultured for 24 hours in complete medium [Eagle's MEM (Univ. of Calif. at San Francisco Cell Culture Facility, San Francisco, CA) supplemented with 10% FCS (Sigma), nonessential amino acids, MEM vitamin solution, L-glutamine, sodium pyruvate, gentamicin (all from Irvine Scientific, Santa Ana, CA) and 7.5% sodium bicarbonate (Sigma)] at 37°C. Selection medium [complete medium containing 1 mg/ml Geneticin (G418 sulfate, GIBCO, Grand Island, NY)]. After 14 days of culture in the selection medium, drug resistant cells were pooled and used in subsequent experiments as a polyclonal population referred to as V51BLim10.

V51BLim10 tumor cells were maintained in Eagle's MEM (Univ. of Calif. at San Francisco Cell Culture Facility, San Francisco, CA) supplemented with 10% FCS (Sigma), non-essential amino acids, MEM vitamin solution, L-glutamine, sodium pyruvate, gentamicin, penicillin- streptomycin (all from Irvine Scientific, Santa Ana, CA) and 1 mg/ml Geneticin. Cell cultures were established from low passage (i.e, less than 10 passages) frozen aliquots and maintained in culture for no more than 30 days prior to use.

V5 1BLim10 cells and the parental 51BLim 10 cells were found to exhibit similar *in vitro* and *in vivo* growth rates. The expression of the neomycin resistance gene in the V51 BLim 1O cells and a variety of other tumor cell lines has had no effect on the tumorigenicity or growth rate of tumors from the injected cells.

### b) Injection of Mice with V51BLim10 Tumor Cells and Monoclonal Antibodies.

The V51BLim10 tumor cells were harvested from tissue culture plates with trypsin-EDTA (Sigma), washed three times in serum-free media (Eagle's MEM) and suspended at a concentration of 2 x 10⁷ cells/ml.

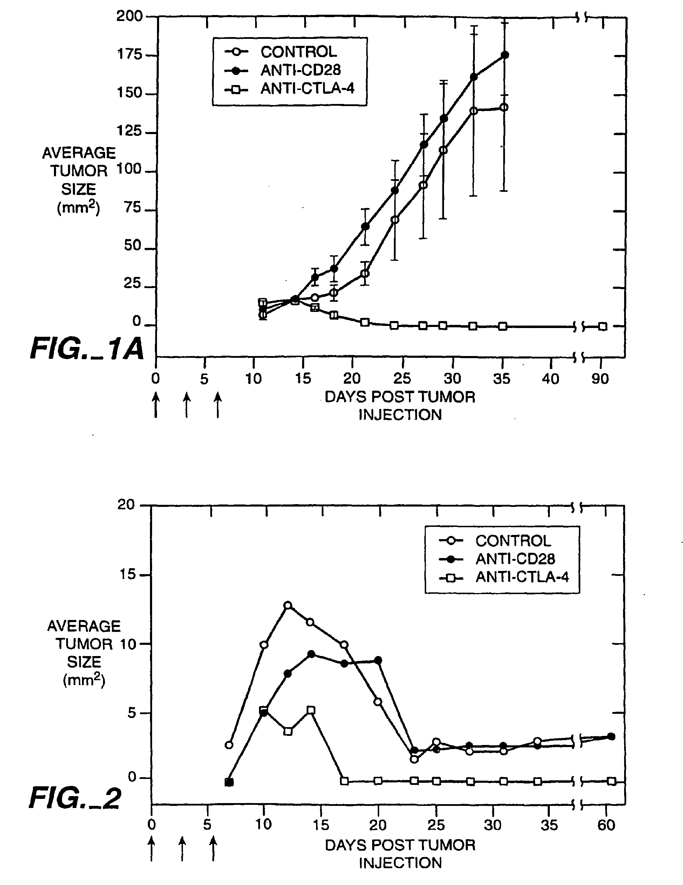

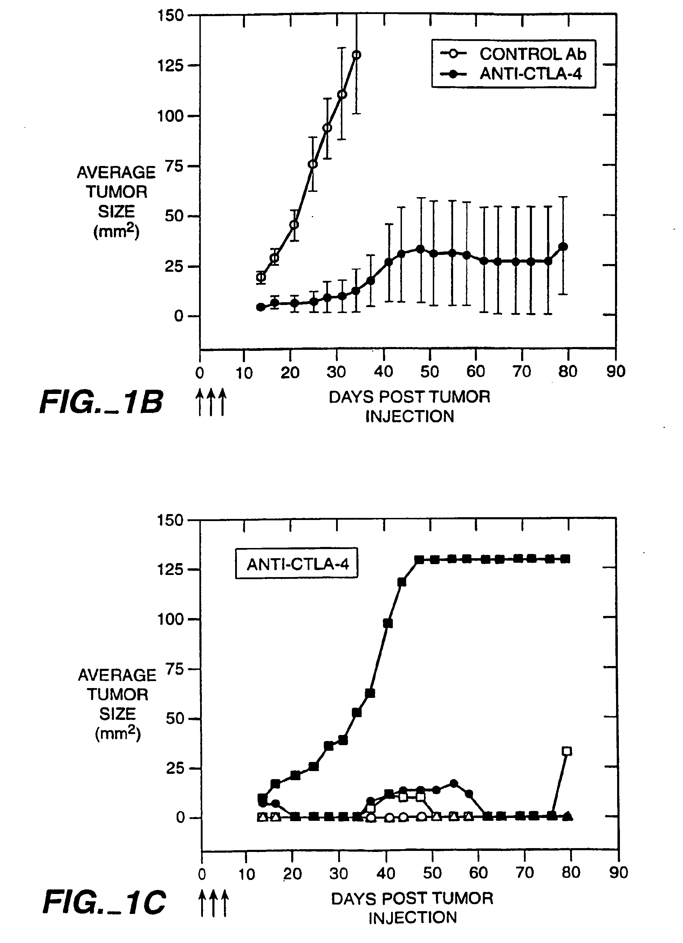

The mice used in this experiment were 6-8 week old female BALB/c mice (Charles River Laboratories, Wilmington, MA). Groups of five mice were anesthetized by methoxyflurane inhalation, ear notched for identification, and injected with 200 µl of the V51BLim10 tumor cell suspension (4 x 10⁶) subcutaneously in the left flank. Treated groups received 100 µg intraperitoneal injections of the antiCTLA-4 mAb 9H10 described above, or alternatively the anti-CD28 mAb, 37.51, on the same day, and additional 50 µg i.p. injections on days 3 and 6 following the injection of the tumor cells (designated by the darkened arrows in Figure 1). The monoclonal anti-CD28, 37.51, is directed against the mouse CD28 protein [Gross et al., J. Immunol. 149:380 (1992)] and served as a negative control.

The mice were monitored for subcutaneous tumor growth and the bisecting diameters of developing tumors were measured with calipers. All of the mice left untreated, or treated with anti-CD28 antibody, developed progressively growing tumors and required euthanasia by 35 days after inoculation. In contrast, all mice treated with anti-CTLA-4 antibody completely rejected their tumors after a short period of limited growth. As shown in Figure 1A, the average tumor area in mm2 (displayed along the y axis) decreased gradually starting at approximately day 14 post- tumor injection (displayed along the x axis), decreasing to zero at approximately day 24. Anti-CTLA-4 treatment was less effective at smaller tumor doses. Figure 1B shows the average tumor size in mice injected with 2 x 10⁶ tumor cells and treated as described above with anti-CTLA-4 antibody or an irrelevent hamster antibody. Anti-CTLA-4 antibody treatment continued to have a dramatic effect on tumor growth, but one mouse developed a tumor quickly, and another much later. Figure 1C illustrates the individual tumor growth in mice injected with 2 x 10⁶ V51BLim10 cells. Three of the mice remained tumor free beyond 80 days. It is clear that CTLA-4 blockade significantly enhanced rejection of the B7 negative tumor cells.

### c) Injection of Mice with B7-51BLim10 Tumor Cells and Monoclonal Antibodies.

51BLim10 cells were transfected as described above, with a plasmid containing the gene for murine B7-1, and cloned by limiting dilution. The B7-51BLim10 tumor cells were harvested from tissue culture plates with trypsin-EDTA (Sigma), washed three times in serum-free media (Eagle's MEM) and suspended at a concentration of 2 x 10⁷ cells/ml.

The mice used in this experiment were 6-8 week old female BALB/c mice (Charles River Laboratories, Wilmington, MA). Groups of five mice were anesthetized by methoxyflurane inhalation, ear notched for identification, and injected with 100 µl of the B7-51BLim10 tumor cell suspension (4 x 10⁶) subcutaneously in the left flank. Treated groups received 100 µg intraperitoneal injections of the antiCTLA-4 mAb 9H10 described above, or alternatively the anti-CD28 mAb, 37.51. Injections of 100, 50 and 50 µg were given on days 0. 3 and 6, respectively (injection days are designated by the darkened arrows in Figure 2). The monoclonal anti-CD28, 37.51, is directed against the mouse CD28 protein [Gross et al., J. Immunol. 149:380 (1992)] and served as a negative control.

The mice were monitored for subcutaneous tumor growth and the bisecting diameters of developing tumors were measured with calipers. The data from this experiment is shown in Figure 2. Treatment with anti-CTLA-4 antibodies inhibited B7-51BLim10 tumor growth as compared to the anti-CD28 and control groups. All mice in the untreated and anti-CD28 treated groups developed small tumors that grew progressively for five to ten days and then ultimately regressed in eight of the ten mice by about day 23 post injection. The two small tumors that did not regress remained static for over 90 days. In contrast, 3 of the 5 mice treated with anti-CTLA-4 antibody developed very small tumors, and all of these regressed completely by day 17.

### d) Anti-CTLA-4 induced rejection of V51BLim10 tumor cells results in protection against subsequent challenge with wild-type colon carcinoma cells.

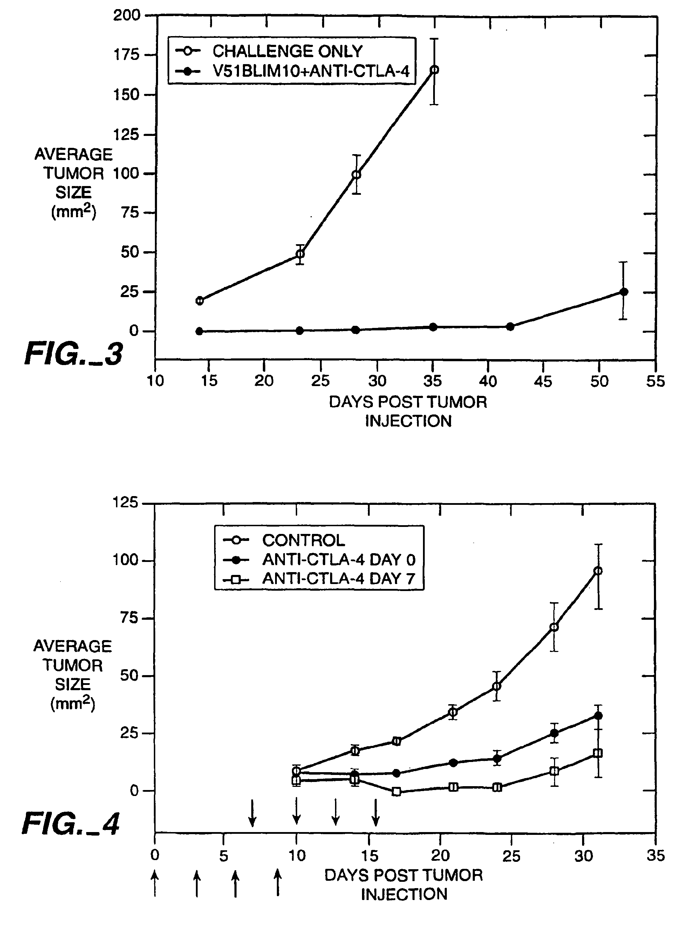

Five anti-CTLA-4 treated mice that had completely rejected V51BLim10 tumor cells were rechallenged 70 days later with 4 x 10⁶ wild-type 51BLim10 tumor cells injected sub-cutaneously in the opposite flank. Five naive mice were also injected as controls. Tumor diameters were measured and reported as described. Prior tumor rejection resulted in significant protection against secondary challenge as compared to naive controls. All control mice developed progressively growing tumors, developed massive tumor burdens, and were euthanized on day 35 post-inoculation. 3 of 5 previously immunized mice remained tumor free 70 days after challenge. Only one of the previously immunized mice had a detectable tumor by day 14, and growth of this tumor was very slow. Utimately, two more tumors developed in the immunized mice 42 days after challenge. The data is shown in Figure 3. These results demonstrated that tumor rejection mediated by CTLA-4 blockade resulted in immunologic memory.

### e) Anti-CTLA-4 treatment reduces the growth of established tumors.

Groups of mice were injected s.c. with 2 x 10⁶ 51BLim10 tumor cells. Control animals (n=10) were injected i.p. with 100 µg irrelevant hamster antibody on days 0, 3, 6 and 9, as indicated by the upward pointing arrows in Figure 4. One anti-CTLA-4 treatment group received i.p. injections on the same days. The other treated mice (n=5) were given i.p. injections of anti-CTLA-4 antibody beginning on day 7 and subsequently on days 10, 13 and 16 (downward pointing arrows). Data is shown in Figure 4. Mice treated with anti-CTLA-4 antibodies at either time point had significantly reduced tumor growth compared to untreated controls. Delaying treatment appeared to be more effective, with 2 of 5 mice remaining tumor free beyond thirty days after inoculation.

### f) Anti-CTLA-4 treatment reduces the growth of the murine fibrosarcoma SA1N.

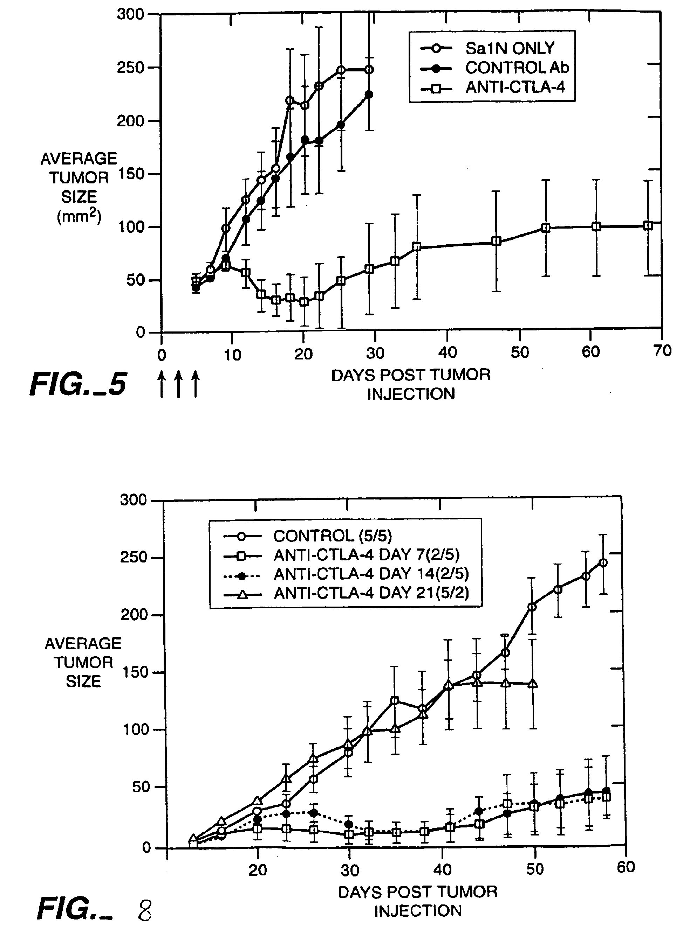

The effects of anti-CTLA-4 treatment were not limited to carcinoma cell lines. Similar results were obtained with a rapidly growing fibrosarcoma cell line of A/JCr mice. Groups of mice were injected s.c. in the flank with a suspension of 1 x 10⁶ SA1N fibrosarcoma cells. Treated groups were injected i.p. with 100 µg anti-CTLA-4 or irrelevant hamster control antibody at days 0, 3 and 6, as indicated by the arrows in Figure 5. All control animals were killed by day 30. Two of five anti-CTLA-4 treated animals remained tumor free at day 55. Data is shown in Figure 5.

### EXAMPLE 3

### Anti-CTLA-4 Monoclonal Antibodies Act as an Adjuvant

### a) Preparation of immunogen

DNP-KLH was obtained from Calbiochem (san Diego, CA) and was suspended in deionized water at 1 mg/ml, 100 ng/ml or 10 pg/ml. One ml of Freund's Complete Adjuvant (Difco, MI) was added to each 1 ml of the DNP-KLH preparations. These were then emulsified in two 5 ml syringes connected by a double-ended luer lock connector by rapid passage through the luer lock, as described in Current Protocols in Immunology, Colligan et al., eds., section 2.4.

30 minutes prior to injection of the immunogen, C57B1/6 mice of 4-6 weeks in age were injected in the peritoneum using a 23 gauge syringe with 200 µg of non-specific control hamster antibody or with 200 µg of anti-CTLA-4 antibody 9H10 (both in 200 µl total volume). The mice were subsequently injected subcutaneously using a 21 gauge syringe at two sites on the back, with 200 µl of the immunogen in the form described above, giving a dose of 100 µg, 10 ng or 1 pg/mouse, respectively. After three days the antibody injections were repeated.

Ten days following the first treatment, the animals were euthanized. Blood was obtained by heart puncture and removed to eppendorf tubes. These samples were allowed to coagulate at 4°C overnight, and were then centrifuged to obtain sera.

Sera was analyzed for isotype specific antibodies recognizing DNP using a standard isotype ELISA, as described in Current Protocols in Immunology (supra.) section 2.1. Briefly, DNP was plated at 100 ng/ml in 50 µl volume in each well of a 96 well Corning modified round-bottom ELISA plate. The wells are blocked using buffers as described. Three-fold serial dilutions of each sera, starting at 1:100 are added to each well. These are incubated for one hour at 25°C, and washed with wash buffer. Isotypes are detected by using mouse specific antibodies as detecting agents at 1 µg/ml in 50 µl of blocking buffer incubated for one hour. The isotype antibodies are biotinylated, and detection is achieved by incubating with avidin-horseradish peroxidase, washing and addition of peroxidase substrate (ABTS, Sigma, Mo.). Stop buffer is added, and the absorbance of each well read with an ELISA reader at a wave length of 490-498 nm within 5-8 min of stopping the reaction.

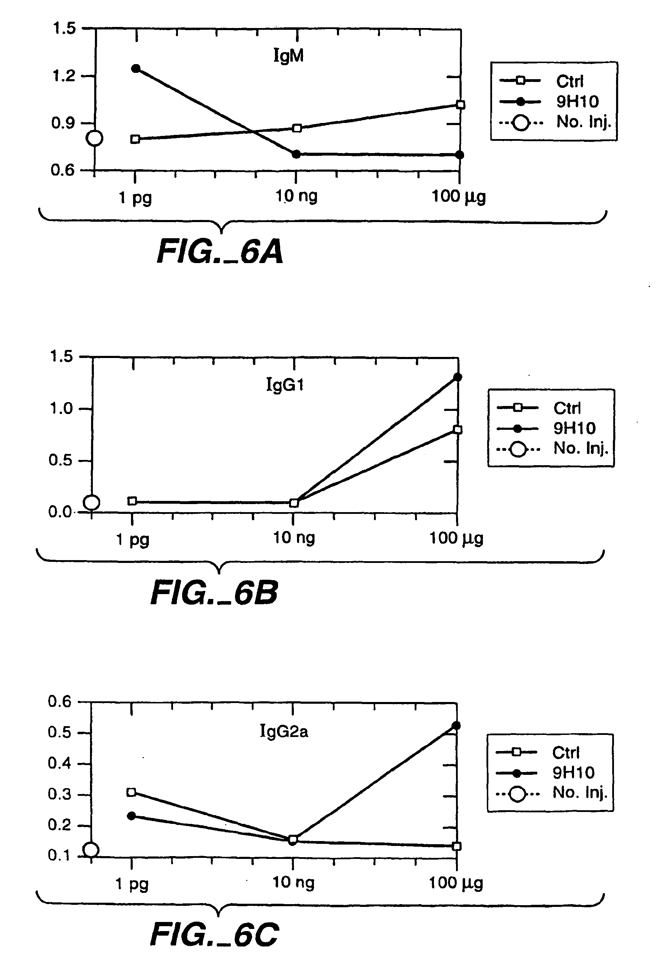

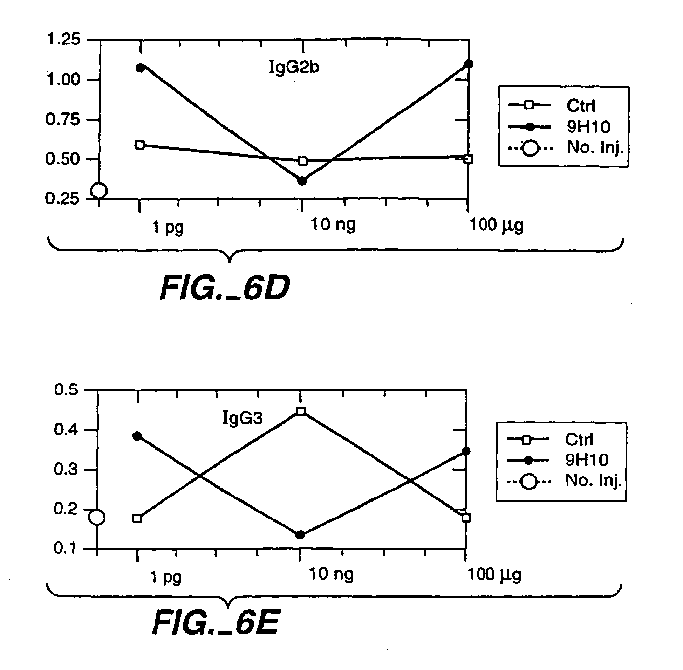

The results are shown in Figures 6A to 6E. Each of the panels illustrates the concentration of a different isotype in the serum sample. The y axis shows the O.D. reading, where an increase in O.D. indicates increased concentration of antibodies in the serum having that isotype. The x axis shows the amount of antigen that was injected, 100 µg, 10 ng or 1 pg per animal, respectively. It can be seen that anti-CTLA-4 antibody increases class switching to IgG1, IgG2a and IgG2b at the higher dose of antigen.

Analysis of T cell function was performed as follows. Lymph node cells were isolated and stimulated in vitro for 72 hours with KLH. The axillary, inguinal, mesenteric, brachial, cervical and popliteal lymph nodes were removed to a dish containing RPMI-complete (10% FCS (Hyclone, Montana), 2 mM glutamine, 50 µM b-mercaptoethanol, 50 µg/ml gentamycin). The lymph nodes were minced to obtain single cell suspensions, filtered through a nytex mesh to remove particulate, and counted using a hemocytometer. Cells were plated in 150 µl of RPMI-complete in 96 well round bottom cluster plates at either 5 x 10⁵, 2.5 x 10⁵, or 1.25 x 10⁵ cells/well. KLH solutions in RPMI-complete were added to final concentrations of 100, 10, 1 or 0 µg/ml and the plates were incubated at 37°C for 64 hours in humidified incubators with 5% CO₂. After 64 hours, 20 µl of RPMI-complete containing 1 µCi of ³H-thymidine was added to each well, and the plates were incubated an additional eight hours. At this time, cultures were harvested onto glass fiber filters using an Inotech 96 well harvester. Filters were dried and counted using a Packard Matrix counter. Each condition was performed in triplicate, and data represents the mean of triplicate values.

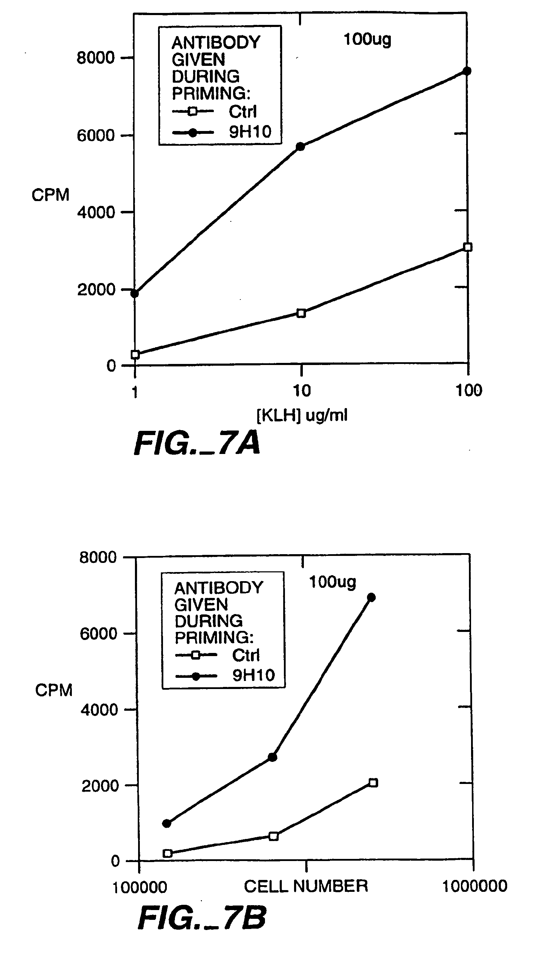

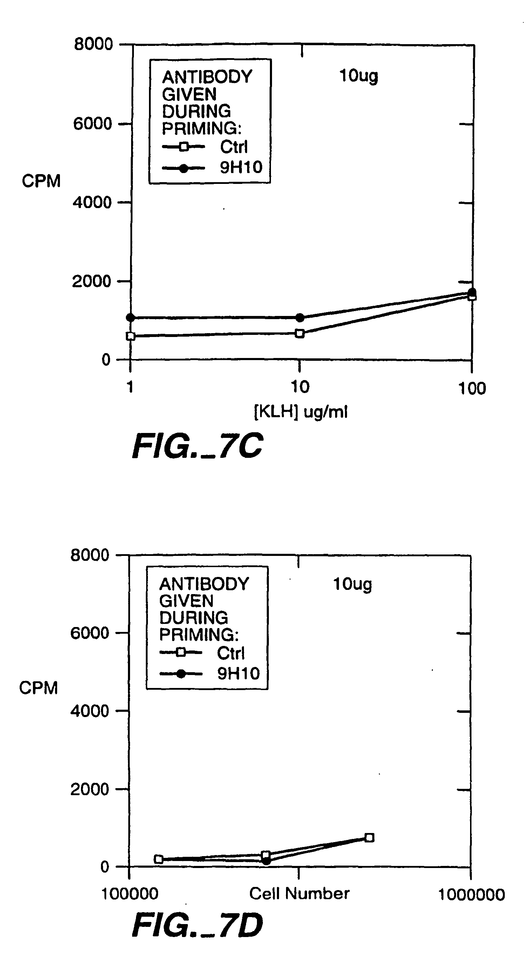

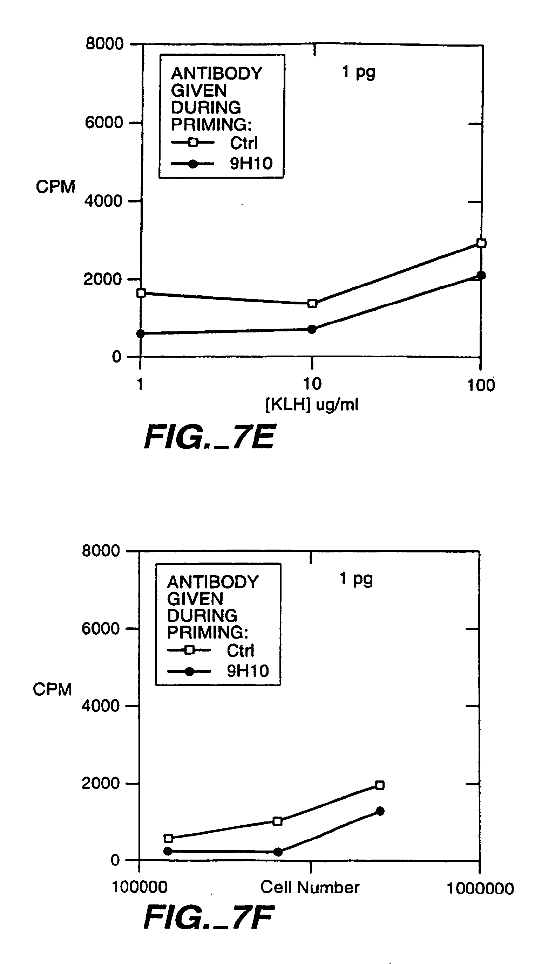

The results are shown in Figures 7A to 7B. The top row shows a constant number of cells (5 x 10⁵ cells), with varying concentrations of antigen (shown on the x axis). The y axis shows incorporation of ³H-thymidine, a measure of cell proliferation. The lower panel shows a constant antigen concentration (10 µg/ml), with varying numbers of cells (shown on the x axis). The data indicates that CTLA-4 blockade strongly upregulates the T cell response to the higher doses of antigen.

The above results demonstrate that the subject treatment with CTLA-4 blocking agents increases the response of T cells to antigenic stimulation. The growth of tumor cells in vivo is greatly diminished in the presence of the subject blocking agents. The effects are observed against unmanipulated, wild-type tumors. CTLA-4 blocking agents not only represent a novel approach to tumor therapy, but, by removing potentially competing inhibitory signals, may be a particularly useful adjunct to other therapeutic approaches involving the co-stimulatory pathway. Class switching by immunoglobulin producing cells, a measure ofT cell help, is greatly increased. The T cell response to immunization with peptide antigens is also greatly increased by the treatment with the subject agents.

### EXAMPLE 4

### Generation of Antibodies Directed Against Human CTLA-4 Proteins

Anti-human CTLA-4 antibodies are generated as follows.

### a) Human CTLA-4 Proteins for Immunization of Host Animals

Immunogens comprising human CTLA-4 proteins contain all or a portion of the extracellular domain of the human CTLA-4 protein. The extracellular domain of the human CTLA-4 protein comprises amino acid residues 38-161, as listed in the database references.

The human CTLA-4 immunogen comprises the entire human CTLA-4 protein or a fusion protein comprising the extracellular domain of human CTLA-4 and a fusion partner. The immunogen comprises the entire human CTLA-4 protein inserted into the membrane of a cell; the cell expressing human CTLA-4 on the surface is used to immunize a host animal.

Immunogens comprising portions of the human CTLA-4 protein are generated using the PCR to amplify DNA sequences encoding the human CTLA-4 protein from mRNA from H38 cells, an HTLV II-associated leukemia line (R. Gallo, National Cancer Institute). The mRNA is reverse transcribed to generate first strand cDNA. The cDNA is then amplified. These sequences are linked to sequences that encode a fusion partner, as described in Linsley et al. [J. Exp. Med. 174:561 (1991)]. The expression vector encodes a fusion protein termed CTLA4Ig, which comprises (from amino- to carboxy-termini) the signal peptide from oncostatin M, the extracellular domain of human CTLA-4 and the H, CH2 and CH3 domains of human IgG 1. The signal peptide from oncostatin M is used in place of the naturally occurring human CTLA-4 signal peptide. The cysteine residues found in the wild-type hinge domain of the human IgG 1 molecule were mutated to serines in the construction of the vector encoding the CTLA41 g protein (Linsley *et al., supra).*

### b) Immunization of Host Animals With Human CTLA-4 Proteins

To immunize animals with immunogens comprising human CTLA-4 proteins, non-human host animals are employed. The immunogen comprising a human CTLA-4/IgG fusion protein (e.g., CTLA4Ig), is used to coat heat-killed *Staphylococcus* A (StaphA) bacteria cells as described in Example lb. Six week old BALB/c mice are injected in the footpad with 50 µl (packed volume) of heat-killed StaphA bacteria coated with approximately 100 µg of CTLA-4Ig suspended in 0.2 ml of PBS.

A total of five injections are given per mouse. On the day of the final boost and prior to the injection, approximately 100 µl of serum is obtained by intraocular bleeding as described in Example lb. The serum is analyzed in companion to serum obtained by the identical methodology prior to the first injection (ie., pre-immune serum).

A human CTLA-4Ig binding ELISA is utilized to demonstrate the presence of antibody that recognizes the human CTLA-4Ig fusion protein in the post-immunization bleed. The human CTLA-4Ig binding ELISA is conducted as described above in Example 1b with the exception that the ELISA plates are coated with human CTLA-4 protein.

The serum and lymph nodes of the immunized mice containing antibody that recognizes the human CTLA-4Ig fusion protein in the post-immunization bleed at serum dilutions 1000-fold greater than the dilution at which background could be detected are collected. Lymphocytes are prepared from draining lymph nodes in the immunized mice and are then used for the generation of monoclonal antibodies directed against the human CTLA-4 protein as described above in Example lc.

Immunogens comprising transformed cells expressing the human CTLA-4 protein on the cell surface are prepared as follows. Expression vectors encoding the entire human CTLA-4 protein are used to transfect the mouse lymphoma cell line EL4 (ATCC TIB 39). Transfected EL4 cells are injected into mice using 1 x 10⁶ to I x 10⁷ transfected cells/injection. The transfected cells are injected in a solution comprising PBS. The mice may be injected either i.p. or in the hind footpad. When i.p. injections are given, a total of approximately 4 injections are administered. When the footpad is used as the site of injection, a total of approximately 5 injections are administered. Serum is collected from the immunized animals and tested for the presence of antibodies directed against the human CTLA-4 protein using an ELISA as described in Example 1b, with the exception that the plates are coated with human CTLA-4 proteins.

### c) Isolation of Hybridoma Lines Secreting Anti-Human CTLA-4 Antibodies

Lymphocytes are isolated from draining lymph nodes or the spleens of animals immunized with the human CTLA-4 immunogen and fused to P3X3.Ag8.653 cells to generate hybridoma cell lines using the PEG fusion protocol described in Example lc. Culture supernatant from wells containing 1000-5000 cells/well are tested for reactivity to human CTLA-4 and for lack of reactivity to a non-CTLA-4 protein such as human CD4 using an ELISA assay.

Hybridomas from positive wells are repetitively cloned by limiting dilution as described in Example 1c. Hybridoma lines secreting monoclonal antibodies that are reactive against human CTLA-4 proteins but not irrelevant human proteins (e.g., human CD4), and that have the ability to stain cells human CTLA-4 transfectants but not control transfectants are selected for production of anti-human CTLA-4 monoclonal antibodies.

### EXAMPLE 5

### Ex Vivo Stimulation of Tumor Infiltrating Lymphocytes (TILs)

Host cells are stimulated *ex vivo,* allowing them to differentiate into tumor-specific immune effector cells. The cells are then reintroduced into the same host to mediate anticancer therapeutic effects.

### a) Isolation of Tumor-Infiltrating Lymphocytes (TILs)

Tumor-infiltrating lymphocytes are obtained using standard techniques. Solid tumors (freshly resected or cryopreserved) are dispersed into single cell suspensions by overnight enzymatic digestion [e.g., stirring overnight at room temperature in RPMI 1640 medium containing 0.01% hyaluronidase type V, 0.002% DNAse type I, 0.1% collagenase type IV (Sigma, St. Louis), and antibiotics]. Tumor suspensions are then passed over Ficoll-Hypaque gradients (Lymphocyte Separation Medium, Organon Teknika Corp., Durham, NC). The gradient interfaces contain viable tumor cells and mononuclear cells are washed, adjusted to a total cell concentration of 2.5 to 5.0 x 10⁵ cells/ml and cultured in complete medium. Complete medium comprises RPMI 1640 with 10% heat-inactivated type-compatible human serum, penicillin 50 IU/ml and streptomycin 50 µg/ml (Biofluids, Rockville, MD), gentamicin 50 µg/ml (GIBCO Laboratories, Chagrin Falls, OH), amphotericin 250 ng/ml (Funglzone, Squibb, Flow Laboratories, McLean, VA), HEPES buffer 10 mM (Biofluids), and L-glutamine 2 mM (MA Bioproducts, Walkersville, MD). Conditioned medium from 3- to 4-day autologous or allogeneic lymphokine-activated killer (LAK) cell cultures (see below) is added at a final concentration of 20% (v/v). Recombinant IL-2 is added at a final concentration of 1000 U/ml.

Cultures are maintained at 37°C in a 5% CO₂₋humidified atmosphere. Cultures are fed weekly by harvesting, pelletting and resuspending cells at 2.5 x 10⁶ cells/ml in fresh medium. Over an initial period (e.g., 2 to 3 weeks) of culture, the lymphocytes selectively proliferate, while the remaining tumor cells typically disappear completely.

To make LAK cell cultures, peripheral blood lymphocytes (PBL) are obtained from patients or normal donors. After passage over Ficoll-Hypaque gradients, cells are cultured at a concentration of 1 x 10⁶/ml in RPMI 1640 medium with 2% human serum, antibiotics, glutamme, and HEPES buffer. Recombinant IL-2 is added at 1000 U/ml. Cultures are maintained for 3 to 7 days in a humidified 5% CO2 atmosphere at 37°

### b) Ex Vivo Stimulation of TILs

4 x 10⁶ cells, in 2 ml of culture medium containing the anti-CTLA-4 mAbs, are incubated in a well of 24-well plates at 37°C in a 5% CO₂ atmosphere for 2 days. The culture medium comprises RPMI 1640 medium supplemented with 10% heat inactivated fetal calf serum, 0.1 mM nonessential amino acids, I µM sodium pyruvate, 2 mM freshly prepared L-glutamine, 100 µg/ml streptomycin, 100 U/ml penicillin, 501lg/ml gentamicin, 0.5 µg/ml fungizone (all from GIBCO, Grand Island, NY) and 5 x 10⁻⁵ M 2-ME (Sigma). The cells are harvested and washed.

The initially stimulated cells are further cultured at 3 x 10⁵/well in 2 ml of culture media with recombinant human IL-2 (available from Chiron Corp., Emeryville, CA; specific activity of 6 to 8 x 10⁶ U/mg protein; units equivalent to 2-3 International U). After 3 days incubation in IL-2, the cells are collected, washed, counted to determine the degree of proliferation, and resuspended in media suitable for intravenous (i.v.) administration (e.g. physiological buffered saline solutions). Bacterial cultures are performed to determine the existence of bacterial contamination prior to reinfusion of the activated cells.

After the activated TILs have been resuspended in a media suitable for injection, IV access is obtained in the host and the cell suspension is infused. Optionally, the host is treated with agents to promote the *in vivo* function and survival of the stimulated cells (e.g. IL-2). Alternatively, CTLA-4 blocking agents are administered in combination with the activated TILs to enhance stimulation of the autoreactive cells *in vivo.*

### EXAMPLE 6

### Effectiveness Against Established Tumor

SA1 is a fibrosarcoma. As shown in Figure 8 the CTLA-4 blockade using 10µg of anti-CTLA-4 antibody per dose is effective even when delayed 7 or 14 days after tumor implantation. This indicates that CTLA-4 blockade can be effective in the treatment of established tumors.

### EXAMPLE 7

### Synergy With Immune Response Stimulating Agent

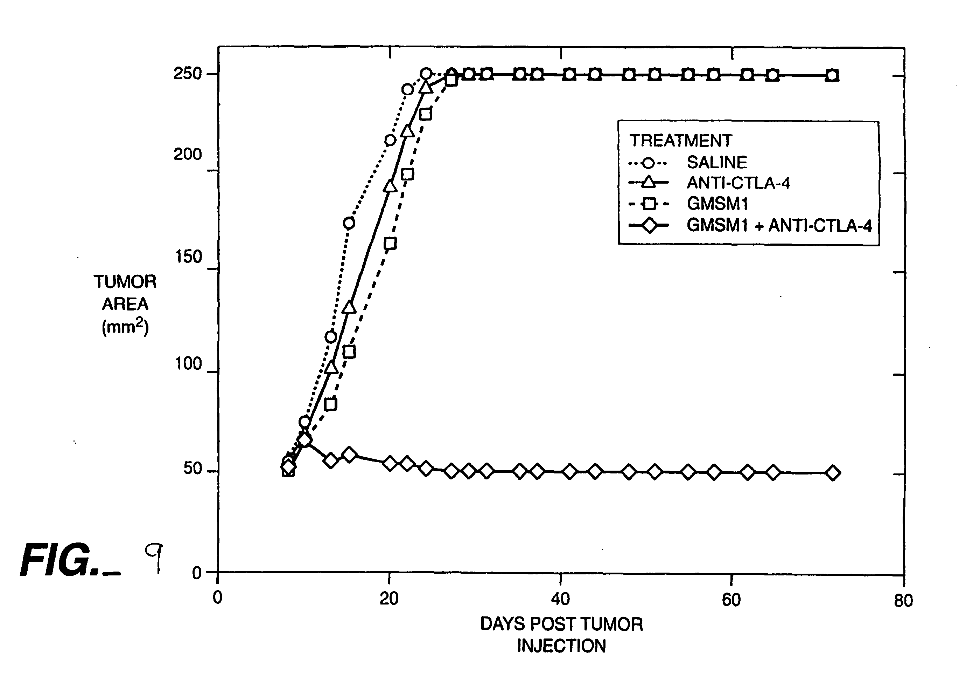

SM1 is a mammary carcinoma that is poorly immunogenic. It is resistant to rejection by transfection with B7. However, some inhibition of growth using B7 and IFNg has been obtained. In the experiment shown in Figure 9, mice received subcutaneous implants of unmodified SM 1 tumor cells, and the indicated treatments on days 0, 3 and 6. As shown, treatment with anti-CTLA-4 (10°µg/dose) by itself had no effect on growth of the tumor. Immunization at a contralateral site with irradiated, GM-CSF transduced cells also had no effect. However, the combination of the two resulted in complete rejection in 4 of 5 mice. This clearly demonstrates that CTLA-4 blockade can synergize with GM-CSF, and probably other lymphokines, to obtain tumor rejection.

### EXAMPLE 8

### Delayed CTLA-4 Blockage

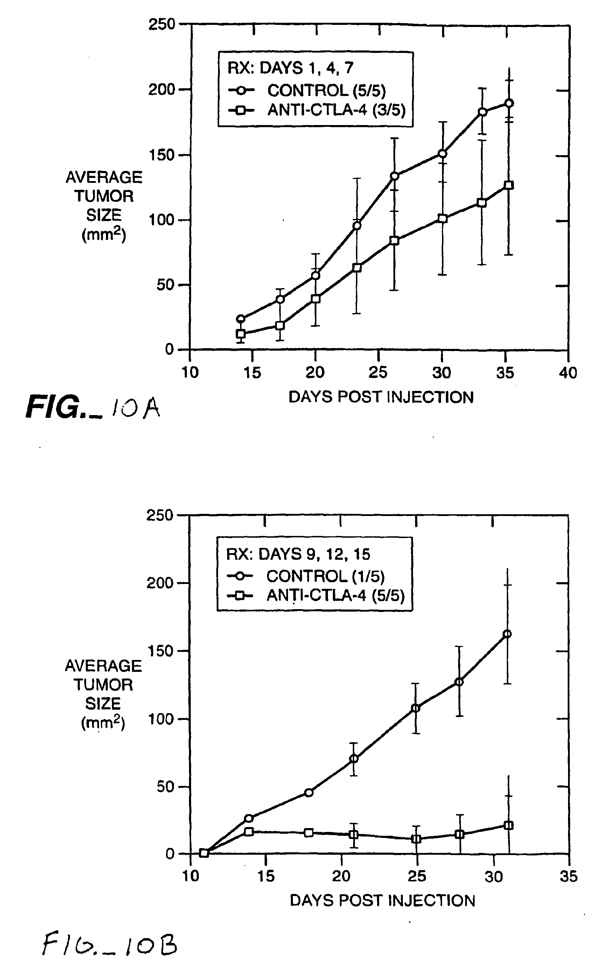

RENCA is a slow growing, poorly immunogenic tumor. As shown in Figures 10A and B, the CTLA-4 blockade (100 µg anti-CTLA-4 antibody per dose) is only poorly effective when initiated at the time of tumor implantation. However, it is quite effective if initiated 9 days after tumor implantation. This suggests that generation of tumor debris from a relatively large tumor mass is important as an agent to stimulate an immune response to obtain effective rejection. This suggests that CTLA-4 blockade could be used at the time of, or shortly after, irradiation or chemotherapy.

### EXAMPLE 9

### CTLA-4 Blockade Enhances Immunogenicity of Tumor Fragments

B16-BL6 was originally derived from the spontaneous murine melanoma cell line B16-F0, by *in vitro* selection for invasive characteristics (Hart, Am. J. Path. 97, no. 3:587-600 (1979)). Both parental line and its variant express low levels of H-2K^{b} and D^{b}, and are negative when stained for MHC class II. Vaccination with irradiated B16-BL6 does not protect against subsequent challenge with live B16-BL6 cells, nor does B7.1 expression result in any significant change in tumor growth *in vivo.*(Chen et al., J. Exp. Med. 179:523-532 (1994), our unpublished results). Consequently, B16-BL6 can be considered to be poorly immunogenic. We have investigated ways of attacking this tumor using CTLA-4 blockade.

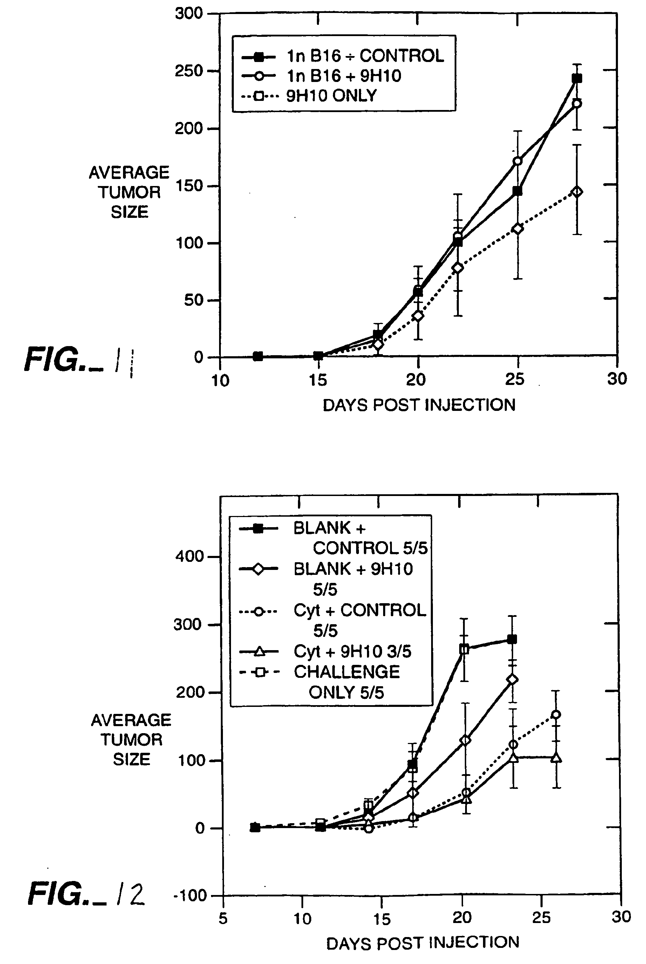

In the experiment shown in Figure 11, mice received subcutaneous implants of unmodified tumor cells and the indicated treatments at days 0, 3 and 6. CTLA-4 blockade by itself (100 µg 9H10/dose) had no effect, nor did immunization with irradiated B 16 cells at a contralateral site. However, treatment with both showed a small, but significant and reproducible inhibition of tumor growth, although no cures were obtained.

This approach was also used in a protective immunization setting. In the experiment shown in Figure 12, mice were immunized with irradiated B 16 cells with and without CTLA-4 blockade (100 µg 9H10/dose) and with and without cytokine-containing gelatin microspheres (containing 50 ng γ interferon and 50 ng GM-CSF). The mice were rechallenged with live, unmodified tumor cells two weeks later. Mice immunized with irradiated cells with CTLA-4 blockade showed significantly impaired tumor growth compared to mice receiving irradiated cells alone. The best protective effect was obtained with cytokine-containing microspheres together with CTLA-4 blockade.

Together, these data indicated that CTLA-4 blockade can enhance immunization strategies employing active immunization with modified tumor cells or tumor fragments, and that it can have a synergistic effect with cytokines.

### EXAMPLE 10

### CTLA-4 Blockade Combined with Tumor Vaccines Can Be Used

### To Stimulate Autoreactive T Cells and Eradicate Poorly-Immunogenic Tumor

In this study the combination of CTLA-4 blockade and GM-CSF producing vaccines was shown to be therapeutically effective against the highly tumorigenic, poorly immunogenic melanoma B 16-BL6, in a mechanism dependent on CD8+ and NK1.1+ cells, but independent of CD4+ T cells. Mice cured from subcutaneous pre-established B 16-BL6 tumors resist rechallenge with B16-BL6 or the parental line B16-F0 after 4 months. Further, B16-F10 induced pulmonary metastases can be eradicated by the combination treatment, and metastatic lesions from these mice show extensive lymphocytic infiltration.

Importantly, in both the subcutaneous and the metastatic melanoma model surviving mice developed skin and hair depigmentation, indicating that autoimmunity directed against pigmented cells was concurrently induced. Since CD4-depleted animals also developed depigmentation it is very likely that this autoimmune phenomenon is induced by cytotoxic T lymphocytes (CTL) directed against pigmentation antigens. This model is well-suited to study the significance of autoreactive CTL in anti-tumor responses, as well as to investigate the role of CTLA-4 in peripheral tolerance in a setting relevant to immunotherapy of cancer.

### Materials and Methods

*Mice:* C57BL/6 female mice (obtained from Charles River Laboratories/NCI) were kept according to institute regulations, and used for tumor experiments when 8-12 weeks old. All subcutaneous injections were done after inhalation of the anaesthetic metoxyflurane.

*Antibodies:* Generation and purification of the hamster anti-murine CTLA-4 antibody 9H10 is described in Example 1 above. Similarly, GK1.5 (anti-CD4), 2.43 (CD8), PK136 (NK1.1), 116.3 (Lyt2.1, Rat IgG, obtained from B. Fowlkes) were prepared in our laboratory as ascites or purified from supernatant using standard procedures. Mouse IgG and hamster IgG were purchased from Jackson Immuno Research Labs (West Grove, PA), Rat IgG was from Sigma (St. Louis, MO). RM4.4-PE (CD4), anti-CD8b2-PE and DX5 (Pharmingen, San Diego, CA) were used to confirm depletions of the relevant populations.

*Cell lines and GM-CSFgene transduction:* B 16-BL6, B16-F10 (obtained from Dr. 1. Fidler, MD Anderson Cancer Center, Houston TX), B 16-F0 (ATCC), and DC2.4 (Shen et al., J. Immunol. 158, no. 6:2723 (1997)) were cultured in DMEM supplemented with 1 U/ml penicillin, 1 µg/ml streptomycin, 50 µg/ml gentamycin, 2 µM L-glutamine, and 8% fetal calf serum (hereafter referred to as complete DMEM). The C57B1/6 derived tumor cell lines EL4 (thymoma) and MC38 (colorectal carcinoma, obtained from Dr. N. Restifo) were maintained in RPMI, supplemented with antibiotics, L-glutamine, 20 µM β-mercaptoethanol, and 8% FCS. GM-CSF producing B16-BL6 and B16-F10 were obtained by retroviral transduction as is known in the art *(see* Dranoff et al., Proc. Natl. Acad. Sci. USA 90:3539-43 (1993). GM-CSF production by short-term lines (F10) or clones (BL6) was tested by sandwich ELISA, using commercially available antibodies to murine GM-CSF (Pharmingen). Clones BL6/GM-E, /GM-18 (producing 5 or 20 ng mGM-CSF/10⁶ cells/24 h), and the short term line F10/GM (30-40 ng/10⁶/24 h) were cultured using complete DMEM. GM-CSF production was routinely confirmed *in vitro* during the course of vaccination experiments. Irradiation of the vaccines before injection enhanced GM-CSF production~1.5-2 fold.

*Subcutaneous challenge and treatment experiments:* Mice were shaved on the back and challenged subcutaneously (s.c.) with 10⁴ B 16-BL6 cells in PBS. At the same day or later as indicated, treatment was initiated by injecting 10⁶ irradiated (16,000 Rad) GM-CSF producing cells (in PBS) s.c. into the left flank, and repeated 3 and 6 days later as indicated. The vaccine consisted of a 1:1 mixture of clones BL6/GM-E and BL6/GM-18. Treatment with 9H10 or control hamster IgG was started simultaneously or three days later with similar results. Antibodies were delivered intraperitoneally (i.p.) at 100 µg in PBS, usually followed by two injections of 50 µg every 3 days. Tumor growth was scored twice to three times per week by measuring perpendicular diameters. Following institute regulations, mice were euthanized when the tumors displayed severe ulceration or reached a size of 300 mm². Depletion of T or NK cells was done by injection of the relevant antibodies (500 µg i.p.) 7, 6, and 5 days prior to challenge, and maintained by repeated injections every ten days during the experiment. Depletions were confirmed in lymph nodes and spleens, 1 day before challenge by flow cytometry using non-crossblocking antibodies. Routinely, < 1% CD4+ cells, CD8+ T cells or NK1.1+ cells were detected in lymph nodes (after CD4 or CD8 depletion) or spleens (NK1.1 depletion) in the respective mice, whereas mice treatment with control antibodies (mouse IgG, rat IgG, or 116.3) demonstrated unchanged lymphocytic profiles as compared to untreated mice.

*Treatment of lung metastases:* To establish lung metastases, mice were injected i.v. with 5x 10⁴ or 10⁵ B 16-F10 cells. Treatment using irradiated F10/GM cells and antibodies was started after 24 hours, following the same protocol as outlined for treatment of subcutaneous tumors. After 25 days, lungs were harvested from each treatment group and surface metastases were counted using a dissection microscope. Paraffin embedded lung section were stained with Hematoxylin-Eosin using standard procedures. For survival experiments, 5x 10⁴ B16-F10 cells were injected i.v. and treatment was started the next day.

*Generation of CTL cultures and IFNγ release assay:* Spleens were harvested from mice rejecting B16-BL6 and restimulated in vitro with B16-BL6/B7.1 or a mixture of B16-F0 and the dendritic cell line DC2.4 after o/n coculture. 5x 10⁶ spleen cells were miced with 10⁵ irradiated (16,000 rad) stimulator cells and recombinant human IL2 was added to a final concentration of 30 IU/ml. After 7 days, cells were collected and purified by Histopaque gradient centrifugation. Live cells (2.5x 10⁵ per well) were stimulated with target cells (5x 10⁴ per well) in 96-well round-bottom plates for 24 hours, after which supernatant was collected and tested for the presence of IFNγ by sandwich Elisa.

### Results

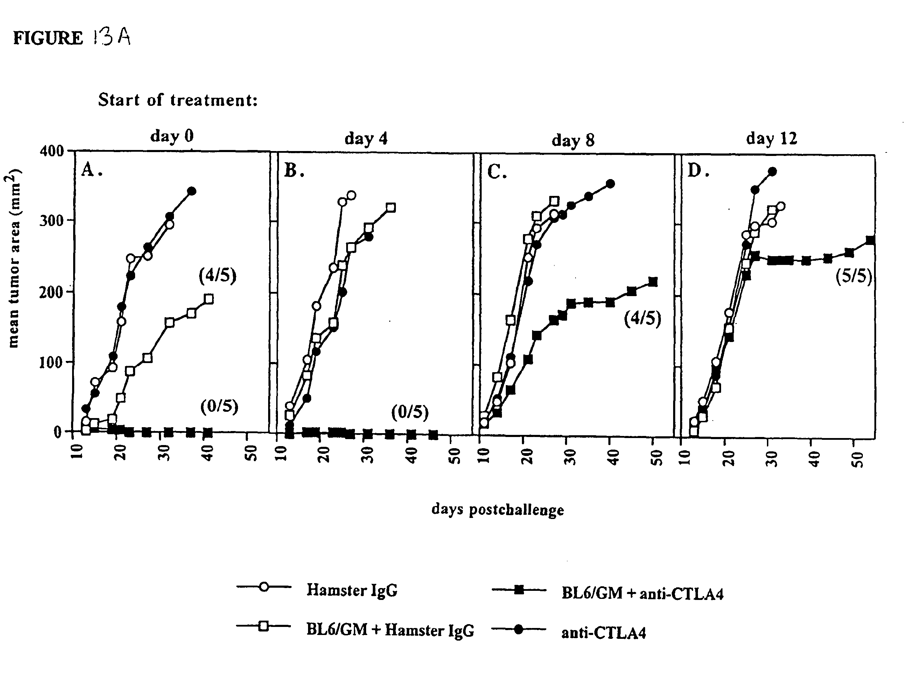

*CTLA-4 blockade together with GM-CSF producing cellular vaccines cause rejection of established B16BL6 tumors.* Hypothesizing that presentation of tumor-associated antigens might be limiting, CTLA-4 blockade was combined with irradiated GM-CSF producing B 16-BL6 cells as an optimal source of antigen. C57BL/6 mice were challenged with 10⁴ B16-BL6 cells subcutaneously and subsequently treated starting on the same day or 4-12 days later. A representative experiment is shown in Figure 13A. As expected, injection of CTLA-4 blocking antibody 9H 10 or control hamster IgG did not induce rejection of pre-established B16-BL6 tumors. When combined with control hamster IgG, vaccination with irradiated GM-CSF producing B 16-BL6 cells demonstrated little or no effect on outgrowth of pre-established tumors. However, combination of GM-CSF producing vaccine and CTLA-4 blockade induced rejection of all tumors injected the same day or 4 days. One of five mice carrying a day 8 B16-BL6 tumor rejected a small palpable tumor after combination treatment including CTLA-4 blockade. No significant effect was found on tumors established 12 days earlier. All experiments combined, an overall success rate of combination treatment of 80% was achieved (68 of 85 mice cured from day 0 or day 4 B 16-BL6 tumors). These results corroborate the finding in Example 7 that CTLA-4 blockade and GM-CSF producing vaccines act synergistically to cause rejection of poorly immunogenic tumors.

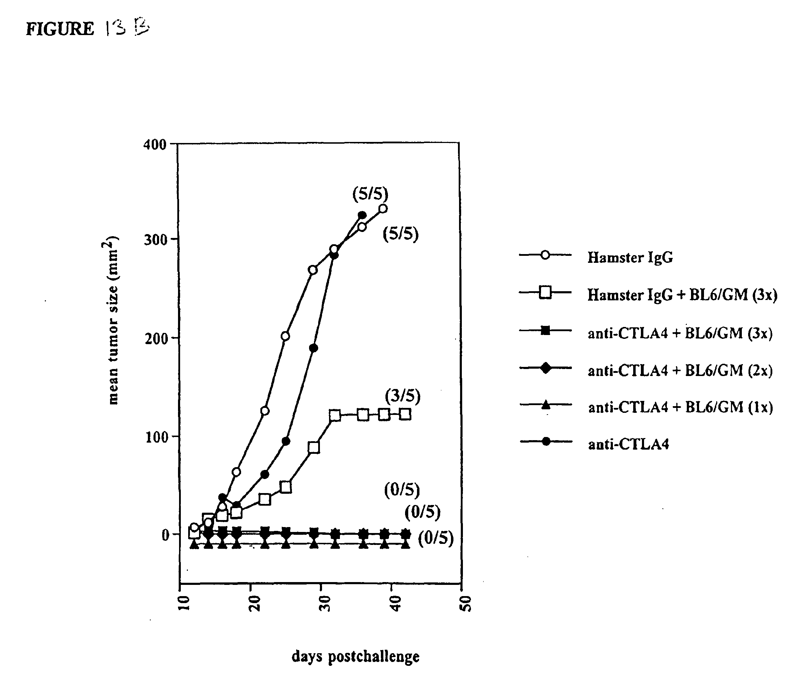

Decreasing the number of vaccinations, we found that a single dose of GM-CSF producing vaccine administered on the same day as tumor challenge was sufficient to eradicate tumors in all the mice when combined with CTLA-4 blockade (Figure 13B). Similarly, a single dose of anti-CTLA-4 following three vaccinations with GM-CSF producing cells was sufficient to induce B16-BL6 rejection (not shown). GM-CSF production by the vaccine was found to be critical for the synergistic effect, since vaccination with irradiated untransduced B 16-BL6 cells in combination with anti-CTLA-4 antibodies was not effective (data not shown).

*Combination of CTLA-4 blockade and GM-CSF producing vaccines induces effective resistance to rechallenge with B16-BL6.* To determine whether mice cured from the initial challenge of B 16-BL6 had developed resistance to rechal lenge, surviving mice received a second challenge of 2 x 10⁴ B16-BL6 on the left flank 128 days after the primary challenge. Also, resistance to the B 16-F0 parental melanoma cell line was tested by injecting 2 x 10⁴ into the right flank. Naive age-matched control mice grew both tumors and were euthanized within 30 days. All mice cured from a primary challenge with B16-BL6, regardless of CTLA4 blockade, rejected B 16-F0, whereas 7 of 9 that received BL6/GM vaccine plus anti-CTLA4 also rejected B16-BL6 (Table I below).

**Table I. Rejection of pre-established B16-BL6 by anti-CTLA-4 and BL6/GM vaccine induces resistance to rechallenge with B16-BL6 and parental B 16-F0.**

| Primary challenge (10⁴B16-B16) treated with: | Tumor incidence at secondary challenge (day 128 after primary): | |
|---|---|---|
| | B16-BL6¹ | B16-F0² |
| anti-CTLA-4+BL6/GM vaccination³ | 2/9⁴ | 0/9 |
| hamster IgG + BL6/GM vaccination | 2/2 | 0/2 |
| control | 5/5 | 5/5 |

| | | |
|---|---|---|
| 1) 2x10⁴ B16-BL6 cells injected s.c. in ten flank. 2) 2x10⁴ B16-F0 cells injected s.c. in right flank. 3) Mice surviving primary challenge with B 16-BL6 after injection of 9H 10 and BL6/GM vaccine on days 0. 3, and 6. 4) Fraction of survivors growing the secondary challenge. | | |

Within the experiment, the two mice that had rejected the primary challenge after BL6/GM vaccination alone were unable to reject a secondary B16-BL6 challenge (Table I). In contrast, 7 or 9 mice that received BL6/GM vaccine plus anti-CTLA-4 also rejected B16-BL6. In two rechallenge experiments, 20 of 24 mice cured from B16-BL6 by combination treatment were immune to secondary challenge with B16-BL6, and 11 mice were resistant to rechallenge with B16-F0. Only 4 of 8 mice cured upon vaccination with GM-CSF-producing cells alone were resistant to rechallenge. Although resistance to rechallenge with B16-BL6 was not found in 100% of the mice, the fact that all mice did reject B16-F0 suggests that mice surviving a primary challenge with B16-BL6 had mounted adequate memory to an antigen(s) shared between parental line and its more invasive variant.

*CD8+ and NKI. 1*+ *cells are required for combination treatment of B16-BL6.* To evaluate whether T and NK cells were involved in the rejection of B 16-BL6, mice were depleted of CD4+, CD8+, or NK 1.1 + cells prior to challenge with B16-BL6. Treatment was started on the same day as tumor implantation following the general schedule of three simultaneous injections of vaccine and anti-CTLA-4. Depletion of CD8+ cells abrogated the effect of treatment (Table II). Mice depleted of NK1.1+ cells were also largely unable to reject their tumor (8/10). We observed that the tumor-bearing NK-depleted mice had developed multiple tumors at the site of challenge, suggesting that NK cells could be involved in the first line of defense against the MHC class I^{lo} B16-BL6 challenge by reducing the initially injected tumor load.

**Table II. Involvement of lymphocyte subsets in rejection of B16-BL6 through co-treatment with anti-CTLA-4 and BL6/GM vaccine.**

| Depletion¹ | B16-BL6 tumor take² | Remarks |
|---|---|---|
| CD4 | 2/10³ | depigmentation⁴ (4/8 survivors) |
| CD8 | 9/10 | --- |
| CD4 + CD8 | 5/5 | --- |
| NK1.1 | 8/10 | multiple tumors developed at injection site, no depigmentation |
| Control mouse IgG | 5/10 | depigmentation |
| | | (3/5 survivors) |
| Control rat IgG | 4/10 | depigmentation |
| | | (4/6 survivors) |
| No depletion | 5/10 | depigmentation |
| | | (3/5 survivors) |
| No depletion, no treatment⁵ | 10/10 | --- |

| | | |
|---|---|---|
| ¹⁾ Depletion of lymphocyte subsets was achieved by injecting depleting antibodies GK 1.5 (anti-CD4), 2.43 (CDB), PK136 (NK 1.1) or control antibodies at days -8.-7,-6 and every 7 (GK 1.5) to 10 days thereafter. Depletion was checked at day -1. Results are compiled from two experiments. ²⁾ Live B 16-BL6 challenge at day 0 was followed by anti-CTLA-4 and BL6/GM vaccination on days 0, 3, and 6. ³⁾ Fraction of mice unable to reject the B16-BL6 challenge. ⁴⁾ Depigmentation observed as a side-effect of treatment (see text). ⁵⁾ Non-depleted mice were left untreated after challenge. | | |

Surprisingly, CD4+ T cells were not required for tumor rejection. In fact, 80% of the mice rejected their tumor, under suboptimal conditions where 50-60% of the control groups rejected B16-BL6 (Table II). Depletion of both CD4+ and CD8+ cells abolished the therapeutic effect. It is apparent that CD8+ T cells and NK1.1+ cells are necessary for rejection of B16-BL6 using CTLA-4 blockade and GM-CSF producing vaccines. Activation of CD8+ T cells involved in the rejection of B16-BL6 melanoma does not appear to be dependent on CD4 help.

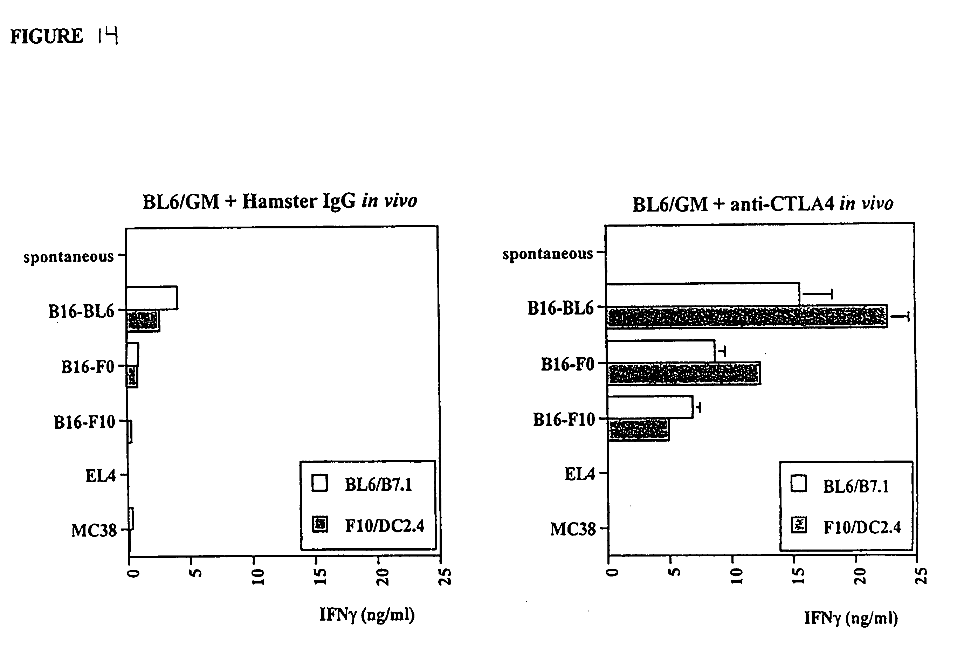

*CTL activity against B16-BL6 is strongly enhanced by CTLA-4 blockade in vivo.* To determine if tumor-reactive CTL were induced by the combination therapy, mice were immunized with BL6/GM plus anti-CTLA-4 or control IgG, and challenged with B 16-BL6 after 4 weeks. Ten days postchallenge, spleens from four mice in each group were pooled and restimulated with B16-BL6/B7.1, or a mixture of B16-F10 and an immortalized dendritic cell line DC2.4. After one round of restimulation in vitro, specific IFNγ release was tested against different variants of B 16 and two unrelated tumor cell lines expressing the H-2^{b} haplotype, the thymoma EL4 and the colorectal carcinoma MC38. As shown in Figure 14, T cells from mice vaccinated with BL6/GM in the presence of control hamster IgG produced very low levels of detectable IFNγ production in this assay, whereas T cells from mice treated with anti-CTLA-4 *in vivo* had greatly enhanced B16-specific IFNγ secretion.

These results indicate that CTLA-4 blockade during vaccination with BL6/GM specifically enhances reactivity towards an antigen (or antigens) expressed by B16 and its variants. In addition, all splenocyte cultures established from mice that were long-term (3-10 months) survivors after combination treatment were found to specifically react with B16 and its variants, as tested by IFNγ release after one round of restimulation *in vitro* (data not shown). Successful rejection of B16-BL6 coincides with the generation of tumor-specific T cell activity.

*Suppression of B16-F10 lung metastases and long-term cure by combination treatment.* To test whether anti-CTLA-4 combined with vaccination would be effective against metastatic disease, 10⁵ B16-F10 cells (selected for metastasis exclusively to the lungs; Fidler, Cancer Res. 35:218-234 (1975)) were injected intravenously and treatment was started one day later. At day 25, mice were sacrificed and surface lung metastases were counted. Treatment with anti-CTLA-4 alone did not show appreciable effect on lung metastasis count compared to control IgG (Table III below). Immunization with F10/GM reduced the number of metastases in a few mice and in combination with anti-CTLA4 further suppressed lung colonization and completely inhibited pulmonary metastases in two of five mice sampled.

**Table III. Reduced number of B16-F10 lung metastases following combination treatment with anti-CTLA-4 and F10/GM vaccine.**

| Treatment of lung metastases:¹ | Lung metastasis count:² |
|---|---|
| Control hamster IgG | >200, >200, >200, 25, 16 |
| Anti-CTLA-4 | >200, >200, >200, >200, >200 |
| Hamster IgG + F10/GM vaccine | >200, >200, 35, 49, 4 |
| Anti-CTLA-4 + F10/GM vaccine | 87, 28, 6, 0, 0 |

| | |
|---|---|
| ¹⁾ B16-F10 (10', i.v.) induced lung metastases were treated with hamster IgG. 9H10, and F10/GM vaccine in combination with either antibody on days 1, 4, and 7 post-challenge. ²⁾ Surface lung metastases were counted under a dissecting microscope. Counts are shown for each individual mouse, all counts over 200 were scored as >200. | |

Histological analysis of these lung samples demonstrated that CTLA-4 blockade in combination with F 10/GM vaccination was associated with infiltration of mononuclear cells in all the metastases stained and observed in 3 of the 5 tumor bearing lungs (the two remaining sets of lungs were found to be tumor-free). Neither anti-CTLA-4 nor F10/GM vaccination alone resulted in lymphocytic infiltration in lung tumors or surrounding tissue. A few polymorphonuclear cells were observed in the smaller metastases from mice vaccinated with F10/GM in the presence of control IgG, but no extensive infiltrate in larger lesions in any of the control groups.

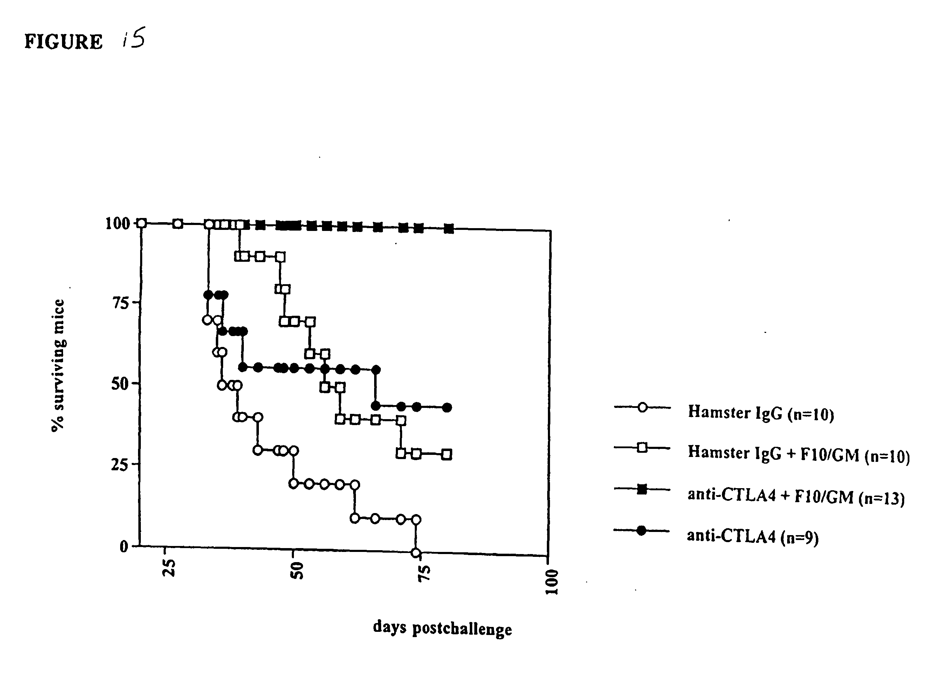

Clearance of lung metastases by combination treatment was confirmed in a survival experiment (Figure 15). Mice challenged with 5x 10⁴ B16-F10 cells, and treated with control hamster IgG all (10/10) succumbed to lung failure due to extensive metastatic disease by day 75 post-injection. Anti-CTLA-4 by itself prolonged survival as did vaccination with F10/GM. However, 13 of 13 mice receiving the combination treatment survived by day 80, and appeared to be cured (Figure 15). Thus, CTLA-4 blockade *in vivo* is therapeutically effective against disseminated disease.

*Mice surviving subcutaneous B16-BL6 tumors or B16-F10 lung metastases develop skin and hair depigmentation.* Within 4 to 8 weeks post-challenge, 56% (38 of 68 cured mice) of the surviving mice developed depigmentation of the skin and hair, starting at the sites of vaccination (left flank) and challenge (back). Moreover, depigmentation was observed at the site of vaccination in a similar proportion of mice surviving B16-F10 lung metastases. Rejection of a B16-BL6 tumor established 8 days before start of treatment induced fast and progressive depigmentation appearing within 25 days post-challenge and spreading to distant sites, indicating that a relatively strong anti-tumor response resulted in rapid manifestation of progressive depigmentation. Depigmentation did occur in mice that received combination treatment in a prophylactic setting, but at a reduced frequency. Interestingly, depigmentation was not dependent on the presence of CD4+ T cells, since 4 of 8 CD4 depleted mice rejecting their tumor also developed progressive depigmentation (Table II, not shown). In some cases, tumor-bearing mice (moribund despite treatment with anti-CTLA4 and BL6/GM) were found to develop small areas of hair depigmentation at the site of progressive tumor growth. Depigmentation was never observed in the mice that were treated by BL6/GM-CSF vaccination without CTLA-4 blockade, or in any of the other treatment groups. These findings suggest that CTLA-4 blockade allows for the activation of autoreactive lymphoid cells that are specifically involved in rejection of a tumor derived from the melanocytic lineage, and may also mediate rejection of normal pigment-containing cells in the skin and hair follicles expressing pigmentation antigens.

The foregoing data demonstrates that combination treatment of the highly tumorigenic, poorly immunogenic melanoma B16-BL6 resulted in overall cure rate of 80%, and that enhancing T cell activation at early stages of tumor growth *in vivo* could induce rejection of primary and secondary challenge with B16-BL6, in a CD8+ CTL dependent fashion. Moreover, outgrowth of pre-established B16-F10 pulmonary metastases was suppressed after a similar combination therapy schedule. Lung metastases in particular might be considered a poor source of antigen for induction of anti-tumor responses. Provision of subcutaneous antigen and blockade of CTLA-4 was sufficient to suppress lung metastasis outgrowth and cure all of the mice under the conditions tested, further stressing the validity of this therapeutic approach.

While not bound by theory, the potency of the combination of the vaccine and anti-CTLA-4 antibody can likely be attributed to enhanced cross-priming of T cells by host APC by the vaccine together with a highly potentiated T cell response as a result of the removal of the inhibitory effects of CTLA-4 by antibody blockade. This results in a synergistic enhancement of the T cell response to a level capable of eliminating the preexisting tumor cell mass. This could occur as a consequence of activation of a larger number of naive T cells due to a lowering of the threshold for activation, or a more sustained response due to temporary removal of signals involved in terminating the response. Rejection is accompanied by long-term memory as indicated by the fact that cured mice rejected rechallenge in the absence of treatment four months after the initial treatment.

Following eradication of B16-B16 tumors 56% of the surviving mice developed depigmentation starting at the sites of vaccination and challenge and spreading to distant sites. Loss of coat color indicated that systemic and progressive autoimmunity had developed towards pigment-bearing cells. For human melanoma patients, a good correlation between autoimmune depigmentation and improved clinical response has been documented. Richards et al., J. Clin. Oncol. 10:1338-43 (1992).

In the present invention, depigmentation and tumor rejection both developed without introducing foreign protein sequences, indicating that CTLA-4 blockade allows for (re)activation of tolerized or ignorant immune effector cells recognizing self antigens. The subject treatment provided a successful, long-term cure for this tumor model, as all mice successfully treated in this experiment have now survived in excess of eighteen months with no readily observable adverse effects other than depigmentation.

### EXAMPLE 11

### CTLA-4 Blockade in a Primary Prostate Tumor Model

As demonstrated by the foregoing examples, the administration of anti-CTLA-4 antibodies is sufficient to induce the rejection of newly implanted and in some cases well-established tumors in several transplantable murine tumor systems. The effectiveness of CTLA-4 blockade in these systems appears to be dependent on the inherent immunogenicity of the tumor. While CTLA-4 blockade by itself is not effective in the treatment of poorly immunogenic transplantable tumors such as the mammary carcinoma SMI 23 or the melanoma B16, eradication of these tumors can be achieved when anti-CTLA-4 is administered together with an irradiated tumor cell vaccine expressing GM-CSF. As shown for the first time in Example 10, in the case of the B16 melanoma tumor rejection is regularly accompanied by a progressive depigmentation, resembling the vitiligo that accompanies immunotherapy in many human melanoma patients. This result suggests that in mice, as in man, the anti-melanoma response is at least in part directed to normal melanocyte-specific antigens.

Given the potency of CTLA-4 blockade combined with cell-based vaccines in poorly immunogenic transplantable tumor models, this study examined the effectiveness of this strategy in the treatment of primary prostatic cancer in TRAMP (TRansgenic Adenocarcinoma of Mouse Prostate) mice. In these mice, SV40 T antigen transgene expression is under the transcriptional control of the rat probasin promoter that directs expression of the oncogene to prostatic epithelium in an androgen-regulated manner. Pathogenesis of neoplasia in TRAMP mice mirrors that in man. When transgene expression begins at puberty, male TRAMP mice develop hyperplasia (5-8 weeks of age), frank neoplasia (8-12 weeks) and eventually invasive adenocarcinoma with metastasis to the lungs, lymph nodes and bone (15-20 weeks). Gingrich et al., Prost Cancer and Prost Dis 6:1-6 (1999).

As shown herein, CTLA-4 blockade in combination with irradiated tumor cell vaccines was effective in reducing tumor incidence and severity of prostatic lesions. In addition, there was significant accumulation of inflammatory cells in the prostates of some vaccinated TRAMP mice. Finally, the anti-tumor response is directed in part to antigens expressed by normal prostate, since immunization of non-transgenic mice with GM-CSF-expressing tumor cell vaccines under conditions of CTLA-4 blockade results in marked prostatitis. This study demonstrates the effectiveness of this immunotherapeutic regimen in primary cancer, and indicates that prostatic tumors express tissue-specific antigens that may provide targets for immunotherapy.

### Methods

*Mice:* All animal procedures were performed according NIH guidelines under protocols approved by the University of California Animal Care and Use Committee. TRAMP mice were bred within our colony on a pure C57BL/6 background. For these experiments, TRAMP mice were backcrossed one time with FVB/N mice and screened for the presence of the transgene by PCR as previously described (Greenberg, et al. Proc. Natl. Acad. Sci. USA 92:3439-43 (1995)).

Mice were vaccinated subcutaneously with 1 x 10⁶ cells each of irradiated (12,000 rads) TRAMP-C 1 and TRAMP-C2 or their GM-CSF-transduced derivatives, GMTRAMP-C1/C2. To maximize antigenic challenge, this treatment was repeated two additional times, three days apart. Even days after the initiation of vaccination, mice were injected intraperitoneally with 100 µg of anti-CTLA-4 (clone 9H10, Example 1) or with purified hamster IgG (Jackson Immunoresearch Corp., West Grove, PA). Additional doses of antibody were administered 3 and 6 days after the first treatment. Mice were followed for morbidity and were euthanized when tumor burden exceeded approximately 50 mm in diameter or animal respiration was strained. Mice were euthanized at the indicated age and the prostatic complex microdissected under a stereomicroscope. Tumor incidence was initially assessed at necropsy and confirmed by histopathologic examination.

*Histopathological Analyses:* The prostatic complex was microdissected into the individual lobes and fixed in 10% neutral buffered formalin. Tissues were processed and stained with hematoxylin and eosin for routine histopathologic analyses. TRAMP tissues were examined by light microscopy and scored using the following criteria: Normal epithelium was assigned a score of 1.0; early signs of prostatic intraepithelial neoplasia (PIN) with tufting of the epithelium and increased nucleus:cytoplasm ratio were scored as 2.0; more advanced PIN with noted cribiform structures and increase in mitotic and/or apoptotic figures was scored as 3.0; the loss of interductal spaces and the invasion of basement membranes by neoplastic epithelium was scored as 4.0; total loss of ductal lumens with evidence of adenocarcinoma was scored as 5.0; and sheets of anaplastic tumor cells were scored as 6.0. To generate a score for each animal, the maximum histologic score for the ventral, dorsal or lateral prostate lobes was used to calculate a mean for the treatment group. The predominant peak score for all TRAMP animals was 4.0 with few histologic scores below 3.0.

*Cell Culture:* TRAMP-C cells are early passage (10-15 passages *in vitro),* non-clonal epithelioid tumor cells independently derived from a TRAMP mouse. Cells were propagated in culture using DMEM (Biowhittaker, Walkersville, MD) supplemented to a final concentration of 5 % fetal calf serum (Biowhittaker), 5 % Nu-Serum (Collaborative Biomedical Products, Bedford, MA), 5 µg/ml insulin (Sigma Chemical, St. Louis, MO), and 0.01 µM dihydrotestosterone.

To obtain GM-CSF-expressing lines, cells were infected with a retrovirus containing the mouse *gm-csf gene* driven by the Maloney murine leukemia virus LTR, using the ψCRIP producer line (gift from Somatix, Inc, Alameda, CA). Retrovirus-containing supernatants were added to TRAMP-C cultures and incubated overnight in the presence of 8 µg/ml polybrene (Sigma). GM-CSF production was assayed by ELISA (Pharmingen, San Diego, CA). Both GMTRAMP-C1 and GMTRAMP-C2 secreted GM-CSF at 150-200 ng/ml/1x10⁶ cells/24 hours. Cells used for injection were released from tissue culture dishes with trypsin (BioWhittaker) and washed three times in Hank's balanced salt solution (BioWhittaker). Cells were resuspended at a density of 1x10⁶ cells/ml, irradiated with 12,000 rads using a cesium-source irradiator and injected subcutaneously in a volume of 0.1 ml.

### Results

*Reduction of primary tumor incidence in TRAMP mice following treatment with cell-based vaccines and anti-CTLA-4.* Male TRAMP mice were vaccinated with a combination of irradiated TRAMP-C 1 and TRAMP-C2 (TRAMP-C 1/C2) or TRAMP-C 1/C2 transduced to express the murine *gm-csf* gene (GMTRAMP-C1/C2) at 14-16 weeks of age. Antibody treatment was begun 7 days after vaccination. To obtain an early indication of the effectiveness of the treatments, four mice from each group were euthanized three weeks after commencement of treatment and examined for tumor incidence at gross necropsy and at the microscopic level following microdissection of the prostatic lobes. While there were no significant differences in mean animal or urogenital tract weight between the treatment groups, there was a striking difference in tumor incidence. Irrespective of vaccine, 11 of 12 mice (92%) in the treatment groups receiving control antibody had detectable tumor. In contrast, only 3 of 12 (25%) mice receiving anti-CTLA-4 had detectable tumor.

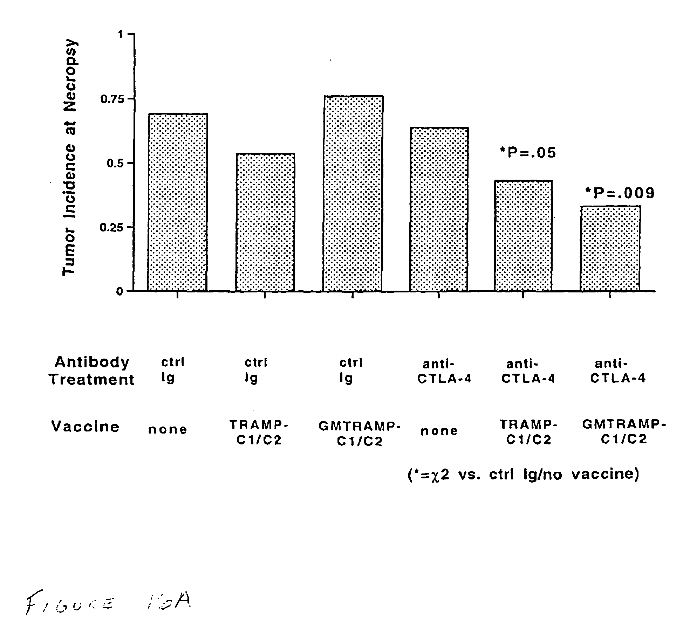

At three weeks after treatment, the tumors in the control antibody-treated mice were sufficiently large to warrant concern about survival of the remaining 150 mice. Therefore, to allow assessment of tumor incidence and tumor grade, the remaining 25 mice in each group were euthanized 5 weeks later (or eight weeks after treatment), and the following criteria assessed at gross necropsy and microdissection of the prostatic complex: animal weight, prostate weight, tumor incidence, and histopathology of prostatic disease. Similar to the analysis at 3 weeks after treatment, there was no significant difference in animal weight or prostate weight between any of the treatment groups. However, there were significant differences in tumor incidence (Figure 16A). A significantly lower tumor incidence was observed in mice treated with anti-CTLA-4 and either the TRAMP-C1/C2 vaccine (43%, P=.05) or the GMTRAMP-C1/C2 vaccine (33%, P=.009) than in mice treated with control antibody alone (69%).

Treatment with anti-CTLA-4 alone had no significant effect on tumor incidence (64%), nor was there significant reduction in tumor incidence in mice receiving the control antibody treatment and either vaccine (55%-TRAMP-C1/C2 and 75%-GMTRAMPC1/C2). Thus, neither CTLA-4 blockade nor vaccination alone was effective at treating primary tumors in TRAMP mice. However, the combination of anti-CTLA-4 and either vaccine had a synergistic effect on tumor incidence. The expression of GM-CSF by the vaccine may potentiate the anti-tumor response since the tumor incidence was slightly lower in mice vaccinated with GMTRAMP-CI/C2 (33% anti-CTLA4+GMTRAMP-C1/C2 versus 43%-anti-CTLA-4+TRAMP-CUC2).

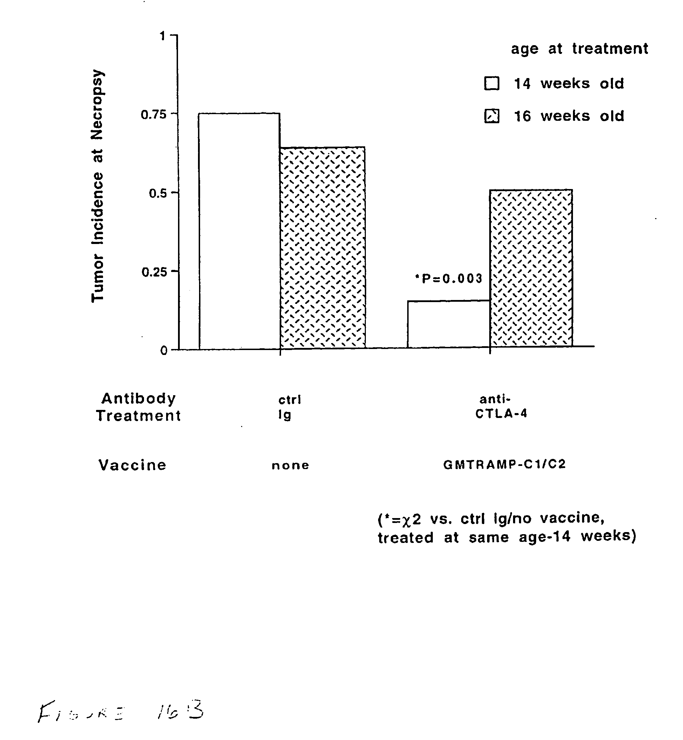

Because each group contained mice from litters with birthdates two weeks apart, tumor incidence was reassessed as a function of age at the initiation of treatment. As shown in Figure 16B for mice vaccinated with GMTRAMP-C1/C2, there was significant reduction in tumor incidence in the mice treated at 14 weeks of age (p=.003), but not in the group treated at 16 weeks of age (p=0.1). This suggests that the stage of tumor development at the time of immunotherapy of TRAMP mice influenced the efficacy of treatment. Tumor incidence in mice treated with TRAMP-C1/C2 and anti-CTLA-4 was equivalent at either age of treatment and was not significantly different from control mice.

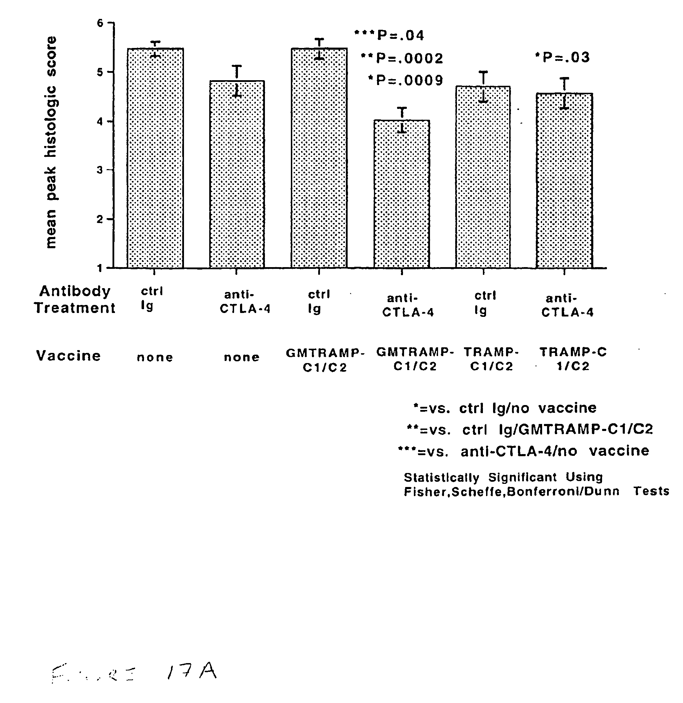

*Reduction of tumor grade in TRAMP mice treated with combination immunotherapy.* To assess the severity of prostate lesions in TRAMP mice, the individual lobes of the prostate were prepared for routine histopathological analysis. A scoring scale was used to evaluate the extent of transformation or tumor grade observed in the prostates of TRAMP mice, as described above. The peak histological score for the ventral, dorsal or lateral prostate lobes was determined for each animal and the average for the treatment group calculated as a mean peak score. As shown in Figure 17A, there was a significant reduction in the severity of lesions in mice treated with anti-CTLA-4 and either vaccine. Specifically, TRAMP mice treated with TRAMP-C1/C2 and anti-CTLA-4 had a significantly lower score (mean peak score 4.6) than control Ig-treated mice (mean peak score 5.5, p=.03). Even more striking was the finding that mice treated with GMTRAMP-CI/C2 and anti-CTLA-4 had a significantly lower tumor grade (mean peak score 3.9) than all three control groups: control Ig/no vaccine (p=.0009), control Ig/GMTRAMP-C1/C2 (mean peak score 5.5. p=.0002), and anti-CTLA-4 treatment alone (mean peak score 4.8, p=.04). Treatment with either vaccine without CTLA-4 blockade or CTLA-4 blockade alone had no significant effect on tumor grade. These findings demonstrate that in addition to reducing the incidence of primary tumors at 8 weeks after treatment, vaccination with a tumor cell-based vaccine in combination with CTLA-4 blockade reduces the severity of prostatic lesions in TRAMP mice.

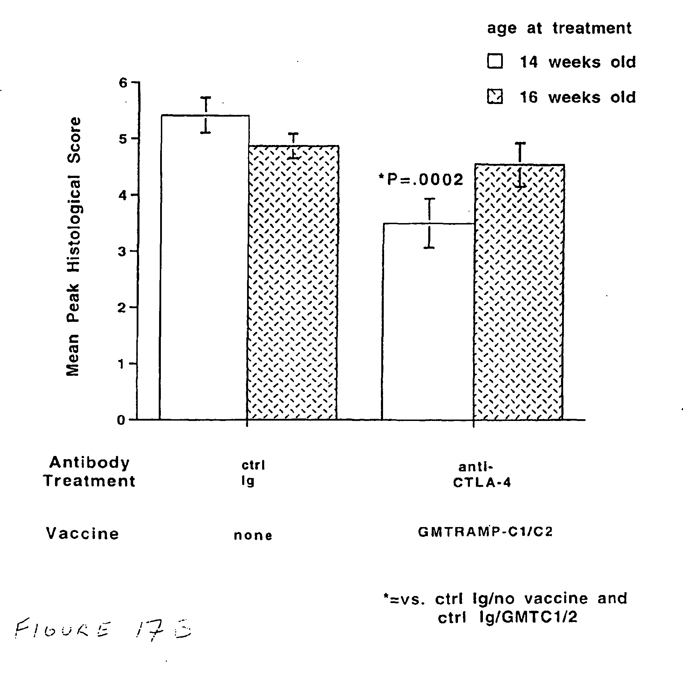

As was performed for analysis of tumor incidence, the histological data was reanalyzed for tumor grade as a function of age at time of treatment. (Figure 17B). Similar to tumor incidence, the highest statistical significance resided in mice treated at 14 weeks of age. Mice treated with GMTRAMP-C1/C2 and antiCTLA-4 (mean peak score=3.5) had a lower tumor grade than mice treated with GMTRAMP-C 1/C2 and control Ig (mean peak score=5.3, p=.0002) and mice treated with control Ig alone (mean peak score=4.7, p=.0002). Interestingly, when treated at 16 weeks of age, TRAMP mice receiving the GMTRAMP-C 1/C2 vaccine and anti-CTLA-4 (mean peak score=4.5) only had a slightly lower mean peak score than mice treated with GMTRAMP-C 1/C2 and control Ig (mean peak score=5.6, p=.03).

Perhaps the most striking histological feature of these analyses was observed in mice treated with GMTRAMP-C1/C2 and anti-CTLA-4, where there was an accumulation of inflammatory cells in the interductal spaces. In these mice, inflammatory cells were closely associated with the vasculature found in the stroma. In contrast, there was no detectable accumulation of inflammatory cells in any of the control Ig-treated mice. In TRAMP mice treated with a GM-CSF-expressing vaccine alone, there were occasional areas where inflammatory cells were detected but these sites were not as extensive as those observed in mice also treated with anti-CTLA-4. The morphological features of the infiltrating cells suggested that the perivascular inflammation was comprised of myeloid as well as lymphoid cells.

*Induction of Prostatitis in Non-Transgenic Mice by Vaccination and CTLA-4 Blockade.* The reduction in incidence and severity of tumors together with the inflammatory infiltrates of the prostate in the TRAMP mice eight weeks after immunization were indicative of a potent immune response. The fact that tumorigenesis in these mice is driven by prostate-specific expression of SV40 Tag raised the possibility that the anti-tumor response was directed against epitopes derived from this viral oncogene. We considered this to be unlikely since TAg expression could not be detected in the vaccine tumor cells by RT-PCR, nor were the tumor cells lysed by CTL reactive against H-2^{b}-restricted epitopes of Tag (personal communication, S. Tevethian and L. Mylin, Pennsylvania State University).

To determine whether the immune response elicited by the therapeutic regimen was limited to oncogene-encoded antigens, non-transgenic C57/BL6 mice were vaccinated and the prostates examined for evidence of inflammation 28 days later. There was no evidence of significant inflammation or tissue damage in the dorsolateral or ventral lobes of the prostates of mice vaccinated with the GMTRAMP-C1/C2 vaccine only. However, there was extensive mononuclear cell infiltration and destruction of glandular epithelium of the male reproductive tract including the dorsolateral prostate in mice vaccinated with GMTRAMP-C1/C2 and treated with anti-CTLA-4. These results demonstrate that the response elicited by the vaccination regimen is directed in part to antigens expressed by normal prostate cells.

The foregoing data demonstrates that CTLA-4 blockade can be synergistically combined with cytokine-expressing, cell-based vaccines to produce an effective treatment regimen for the treatment of primary tumors. The reduction of both tumor incidence as well as histological tumor grade indicates that the combination of a cell-based vaccine together with anti-CTLA-4 was sufficient to slow the progression of primary prostatic tumors. It is not surprising that the immune system is unable to completely eliminate tumors in this aggressive model, but it is remarkable that an anti-tumor immune response can have a significant impact on disease progression in a situation where an entire organ is undergoing transformation. The ability of a cell-based vaccine in combination with CTLA-4-blockade to significantly reduce tumor incidence and burden in the aggressive TRAMP model underscores the remarkable efficacy of this immunotherapeutic approach.

TRAMP mice treated with either the vaccine or antibody alone had no reduction in tumor incidence or tumor grade whereas the combination of both resulted in a significant reduction in both criteria. This suggests that an additional source of antigen from the cell-based vaccine contributes to T cell priming, which is enhanced by blockade of CTLA-4/B7 interactions. The fact that tumor incidence and tumor grade were lower in mice that received the GMTRAMP-C1/C2 vaccine than those receiving the TRAMP-C1/C2 vaccine suggest that the effect is enhanced by the recruitment and activation of APCs by GM-CSF expression.

Vaccination of non-transgenic mice with the same therapeutic strategy demonstrated to be effective for treatment of TRAMP mice led to autoimmune prostatitis and destruction of some prostatic epithelium. This finding suggests that the vaccination approach is capable of inducing an autoimmune response against normal prostate antigens. These results further support the idea that effective tumor immunity is, in fact, closely tied to autoimmunity.

Rather than being viewed as a troublesome side effect of tumor immunotherapy, the intentional induction of autoimmunity to defined tissue-specific antigens can provide a practical strategy for the generation of effective anti-tumor responses. As demonstrated herein, the CLTA-4 blockade of the present invention provides an effective immunological treatment of tumors arising from non-essential tissues.

### EXAMPLE 12

### CTLA-4 Blockade Induces Immunity To Peptides Derived from Normal Melanoma-Specific Proteins

gp100 is a melanocyte-specific protein identified as a frequent target of T cells from human melanoma patients. Previous work had shown that mice could not be immunized with peptides derived from the normal mouse peptides, but that immunity to the mouse peptide could be obtained if mice were immunized with the corresponding human sequence. This suggested that immunization with human broke tolerance to the mouse, allowing the development of autoimmunity. This study was directed to determining whether CTLA-4 blockade could be effective in inducing autoimmunity to the syngeneic mouse peptide,

Splenic dendritic cells (DC) were isolated from C57BL6 mice (H-2b) by conventional techniques. DCs were then incubated with or without an H-2b binding peptide corresponding to the normal mouse gp100 sequence (Mgp100). At day -21 mice were immunized with peptide-pulsed or -unpulsed DC followed by injection of 100µg anti-CTLA-4 (MAb 9H10) or control hamster antibody (MAb 560). At day -14 and -7 the mice were boosted by injection of appropriate DCs, but without any antibody treatment. At day 0 mice from each treatment cohort were separated into two groups for analysis.

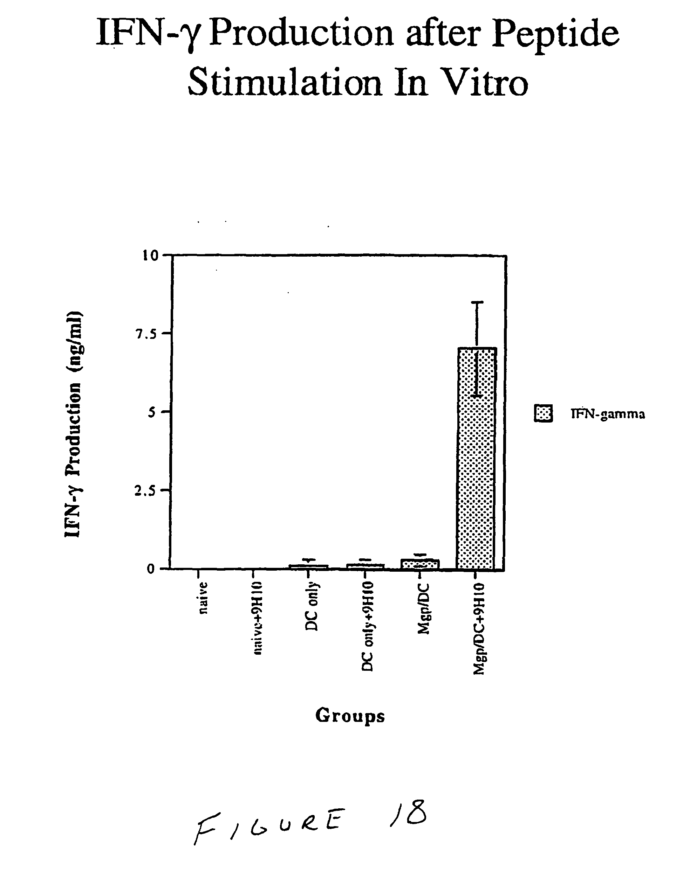

Group A: Lymph node T cells were purified and examined for ability to produce IFNγ in response to stimulation by syngeneic antigen-presenting cells pulsed with Mgp100. As shown In Figure 18; only mice immunized under conditions of CTLA-4 blockade made significant amounts of IFNγ.

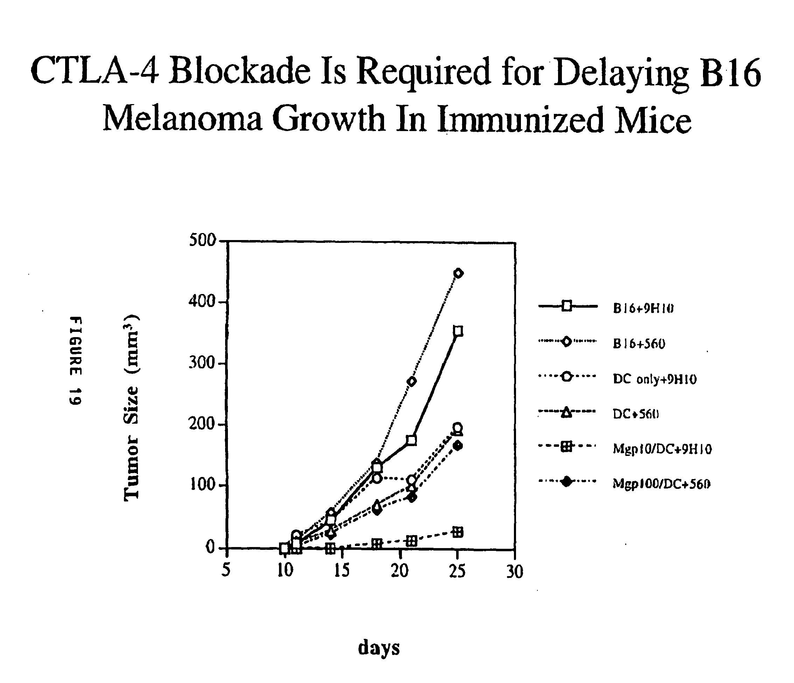

Group B: Mice were challenged with B16 melanoma cells implanted subcutaneously on the back. Tumor growth was assessed by measuring diameter in two dimensions using calipers. As shown in Figure 19, only the group immunized under conditions of CTLA-4 blockade showed inhibition of tumor growth. Although all the mice eventually succumbed to tumor, this result shows the induction of a very effective anti-tumor response.

The foregoing examples provide compelling support for the therapeutic potential of the blockade of inhibitory signal of T cell activation mediated by CTLA-4 as a strategy for enhancing anti-tumor responses by induction of immunity to tissue-specific self antigens.

Although the foregoing invention has been described in some detail by way of illustration and example for purposes of clarity of understanding, it will be readily apparent to those of ordinary skill in the art in light of the teachings of this invention that certain changes and modifications may be made thereto without departing from the scope of the appended claims.

## Claims

1. Use of an anti-CTLA-4 antibody or fragment thereof and a self-antigen preparation for the manufacture of a medicament for treating established metastases.

2. Use according to claim 1, wherein the self-antigen preparation comprises tumor cells.

3. Use according to claim 2, wherein the tumor cells are irradiated tumor cells.

4. Use according to any one of claims 1 to 3, further comprising use of a cytokine in the manufacture of the said medicament.

5. Use according to claim 4, wherein the cytokine is provided as tumor cells transduced with the cytokine.

6. Use according to claim 5, wherein the tumor cells are irradiated cells.

7. Use according to any of claims 4 to 6, wherein the cytokine is GM or GM-CSF.

8. Use according to claim 1, further comprising use of a tumor vaccine in the manufacture of said medicament.

9. Use according to any of claims 1 to 8, wherein the medicament is for treating metastases where loss or modification of some or all of the normal tissue is acceptable.

10. Use according to any of claims 1 to 8, wherein said metastases are pulmonary metastases from a melanoma.

11. Use according to any of claims 1 to 10, wherein the said medicament is for treating a human.

12. Use according to any of claims 1 to 11, wherein the said antibody is a monoclonal antibody.

13. Use according to any of claims 1 to 12, wherein the said antibody is a humanized antibody.

14. An anti-CTLA-4 antibody or fragment thereof for use in treating established metastases in simultaneous or sequential combination with a self-antigen preparation.

15. The antibody or fragment thereof according to claim 14, wherein the self-antigen preparation comprises tumor cells.

16. The antibody or fragment thereof according to claim 15, wherein the tumor cells are irradiated tumor cells.

17. The antibody or fragment thereof according to any of claims 14 to 16, which is for use in treating established metastases in simultaneous or sequential combination with a cytokine in addition to the self antigen preparation.

18. The antibody or fragment thereof according to claim 17, wherein the cytokine is provided as tumor cells transduced with the cytokine.

19. The antibody or fragment thereof according to claim 18, wherein the tumor cells are irradiated cells.

20. The antibody or fragment thereof according to any of claims 17 to 19, wherein the cytokine is GM or GM-CSF.

21. The antibody or fragment thereof according to claim 14, which is for use in simultaneous or sequential combination with a tumor vaccine.

22. The antibody or fragment thereof according to any of claims 14 to 21, which is for use in treating metastases where loss or modification of some or all of the normal tissue is acceptable.

23. The antibody or fragment thereof according to claim 22, which is for use in treating pulmonary metastases from a melanoma.

24. The antibody or fragment thereof according to any of claims 14 to 23, which is for use in treating a human.

25. The antibody or fragment thereof according to any of claims 14 to 24, which is a monoclonal antibody or fragment thereof.

26. The antibody or fragment thereof according to any of claims 14 to 25, which is a humanized antibody or fragment thereof.

27. A self antigen preparation for use as an immune response stimulating agent in the treatment of established metastases in simultaneous or sequential combination with an anti-CTLA-4 antibody or fragment thereof.

## Patentansprüche

1. Verwendung eines Anti-CTLA-4-Antikörpers oder eines Fragments davon sowie einer Selbst-Antigen-Zubereitung zur Herstellung eines Arzneimittels zur Behandlung festgestellter Metastasen.

2. Verwendung nach Anspruch 1, worin die Selbst-Antigen-Zubereitung Tumorzellen umfasst.

3. Verwendung nach Anspruch 2, worin die Tumorzellen bestrahlte Tumorzellen sind.

4. Verwendung nach einem der Ansprüche 1 bis 3, umfassend weiter die Verwendung eines Cytokins bei der Herstellung des Medikaments.

5. Verwendung nach Anspruch 4, worin das Cytokin bereitgestellt wird als Tumorzellen, die mit dem Cytokin transduziert sind.

6. Verwendung nach Anspruch 5, worin die Tumorzellen bestrahlte Zellen sind.

7. Verwendung nach einem der Ansprüche 4 bis 6, worin das Cytokin GM oder GM-CSF ist.

8. Verwendung nach Anspruch 1, umfassend weiter eine Verwendung eines Tumor-Impfstoffs bei der Herstellung des Arzneimittels.

9. Verwendung nach einem der Ansprüche 1 bis 8, worin das Medikament ein Medikament zur Behandlung von Metastasen ist, bei dem ein Verlust oder eine Modifikation eines Teils oder des gesamten normalen Gewebes annehmbar ist.

10. Verwendung nach einem der Ansprüche 1 bis 8, worin die Metastasen pulmonale Metastasen von einem Melanom sind.

11. Verwendung nach einem der Ansprüche 1 bis 10, worin das Arzneimittel ein Arzneimittel zur Behandlung eines Menschen ist.

12. Verwendung nach einem der Ansprüche 1 bis 11, worin der Antikörper ein monoklonaler Antikörper ist.

13. Verwendung nach einem der Ansprüche 1 bis 12, worin der Antikörper ein humanisierter Antikörper ist.

14. Ein Anti-CTLA-4-Antikörper oder ein Fragment davon zur Verwendung bei der Behandlung festgestellter Metastasen in simultaner oder sequentieller Kombination mit einer Selbst-Antigen-Zubereitung.

15. Der Antikörper oder das Fragment davon nach Anspruch 14, worin die Selbst-Antigen-Zubereitung Tumorzellen umfasst.

16. Der Antikörper oder das Fragment davon nach Anspruch 15, worin die Tumorzellen bestrahlte Tumorzellen sind.

17. Der Antikörper oder das Fragment davon nach einem der Ansprüche 14 bis 16, zur Verwendung bei der Behandlung festgestellter Metastasen in simultaner oder sequentieller Kombination mit einem Cytokin zusätzlich zu der Selbst-Antigen-Zubereitung.

18. Der Antikörper oder das Fragment davon nach Anspruch 17, worin das Cytokin bereitgestellt wird als Tumorzellen, die mit dem Cytokin transduziert sind.

19. Der Antikörper oder das Fragment davon nach Anspruch 18, worin die Tumorzellen bestrahlte Zellen sind.

20. Der Antikörper oder das Fragment davon nach einem der Ansprüche 17 bis 19, worin das Cytokin GM oder GM-CSF ist.

21. Der Antikörper oder das Fragment davon nach Anspruch 14, zur Verwendung in simultaner oder sequentieller Kombination mit einem Tumor-Impfstoff.

22. Der Antikörper oder das Fragment davon nach einem der Ansprüche 14 bis 21 zur Verwendung bei der Behandlung von Metastasen, bei der ein Verlust oder eine Modifikation eines Teils oder des gesamten normalen Gewebes annehmbar ist.

23. Der Antikörper oder das Fragment davon nach Anspruch 22, zur Verwendung bei der Behandlung von pulmonalen Metastasen von einem Melanom.

24. Der Antikörper oder das Fragment davon nach einem der Ansprüche 14 bis 23, zur Verwendung bei der Behandlung eines Menschen.

25. Der Antikörper oder das Fragment davon nach einem der Ansprüche 14 bis 24, welcher/welches ein monoklonaler Antikörper oder ein Fragment davon ist.

26. Der Antikörper oder das Fragment davon nach einem der Ansprüche 14 bis 25, welcher/welches ein humanisierter Antikörper oder ein Fragment davon ist.

27. Eine Selbst-Antigen-Zubereitung zur Verwendung als Immunantwort-stimulierendes Mittel bei der Behandlung von festgestellten Metastasen in simultaner oder sequentieller Kombination mit einem Anti-CTLA-4-Antikörper oder einem Fragment davon.

## Revendications

1. Utilisation d'un anticorps anti-CTLA-4 ou d'un de ses fragments et d'une préparation d'auto-antigène pour la production d'un médicament destiné au traitement de métastases établies.

2. Utilisation suivant la revendication 1, dans laquelle la préparation d'auto-antigène comprend des cellules tumorales.

3. Utilisation suivant la revendication 2, dans laquelle les cellules tumorales sont des cellules tumorales irradiées.

4. Utilisation suivant l'une quelconque des revendications 1 à 3, comprenant en outre l'utilisation d'une cytokine dans la production dudit médicament.

5. Utilisation suivant la revendication 4, dans laquelle la cytokine est fournie sous forme de cellules tumorales transduites avec la cytokine.

6. Utilisation suivant la revendication 5, dans laquelle les cellules tumorales sont des cellules irradiées.

7. Utilisation suivant l'une quelconque des revendications 4 à 6, dans laquelle la cytokine est la cytokine GM ou GM-CSF.

8. Utilisation suivant la revendication 1, comprenant en outre l'utilisation d'un vaccin antitumoral dans la production dudit médicament.

9. Utilisation suivant l'une quelconque des revendications 1 à 8, dans laquelle le médicament est destiné au traitement de métastases lorsque la perte ou la modification d'une partie ou de la totalité du tissu normal est acceptable.

10. Utilisation suivant l'une quelconque des revendications 1 à 8, dans laquelle lesdites métastases sont des métastases pulmonaires d'un mélanome.

11. Utilisation suivant l'une quelconque des revendications 1 à 10, dans laquelle ledit médicament est destiné au traitement d'un être humain.

12. Utilisation suivant l'une quelconque des revendications 1 à 11, dans laquelle ledit anticorps est un anticorps monoclonal.

13. Utilisation suivant l'une quelconque des revendications 1 à 12, dans laquelle ledit anticorps est un anticorps humanisé.

14. Anticorps anti-CTLA-4 ou un de ses fragments destiné à être utilisé dans le traitement des métastases établies en association simultanée ou séquentielle avec une préparation d'auto-antigène.

15. Anticorps ou son fragment suivant la revendication 14, dans lequel la préparation d'auto-antigène comprend des cellules tumorales.

16. Anticorps ou son fragment suivant la revendication 15, les cellules tumorales étant des cellules tumorales irradiées.

17. Anticorps ou son fragment suivant l'une quelconque des revendications 14 à 16, qui est destiné à être utilisé dans le traitement de métastases établies en association simultanée ou séquentielle avec une cytokine en plus de la préparation d'auto-antigène.

18. Anticorps ou son fragment suivant la revendication 17, la cytokine étant fournie sous forme de cellules tumorales transduites avec la cytokine.

19. Anticorps ou son fragment suivant la revendication 18, les cellules tumorales étant des cellules irradiées.

20. Anticorps ou son fragment suivant l'une quelconque des revendications 17 à 19, la cytokine étant le GM ou GM-CSF.

21. Anticorps ou son fragment suivant la revendication 14, qui est destiné à être utilisé en association simultanée ou séquentielle avec un vaccin antitumoral.

22. Anticorps ou son fragment suivant l'une quelconque des revendications 14 à 21, qui est destiné à être utilisé dans le traitement de métastases lorsque la perte ou la modification d'une partie ou de la totalité du tissu normal est acceptable.

23. Anticorps ou son fragment suivant la revendication 22, qui est destiné à être utilisé dans le traitement de métastases pulmonaires provenant d'un mélanome.

24. Anticorps ou son fragment suivant l'une quelconque des revendications 14 à 23, qui est destiné à être utilisé dans le traitement d'un être humain.

25. Anticorps ou son fragment suivant l'une quelconque des revendications 14 à 24, qui est un anticorps monoclonal ou son fragment.

26. Anticorps ou son fragment suivant l'une quelconque des revendications 14 à 25, qui est un anticorps humanisé ou son fragment.

27. Préparation d'auto-antigène destinée à être utilisée comme agent stimulant la réponse immunitaire dans le traitement de métastases établies en association simultanée ou séquentielle avec un anticorps anti-CTLA-4 ou son fragment.


### SEQUENCE LISTING

(1) GENERAL INFORMATION:
   (i) APPLICANT: The Regents of the University of California
   (ii) TITLE OF INVENTION: Stimulation of T Cells Against Self Antigens Using CTLA-4 Blocking Agents
   (iii) NUMBER OF SEQUENCES: 2
   (iv) CORRESPONDENCE ADDRESS:
      (A) ADDRESSEE: FLEHR HOHBACH TEST ALBRITTON & HERBERT LLP
      (B) STREET: 4 Embarcadero Center, Suite 3400
      (C) CITY: San Francisco
      (D) STATE: California
      (E) COUNTRY: USA
      (F) ZIP: 94111-4187
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (vi) CURRENT APPLICATION DATA:
      (A) APPLICATION NUMBER: US
   (viii) ATTORNEY/AGENT INFORMATION:
      (A) NAME: Lorenz, Todd A.
      (B) REGISTRATION NUMBER: 39,754
      (C) REFERENCE/DOCKET NUMBER: FP-68668
   (ix) TELECOMMUNICATION INFORMATION:
      (A) TELEPHONE: (415) 781-1989
      (B) TELEFAX: (415) 398-3249
(2) INFORMATION FOR SEQ ID NO:1:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "Primer"
   (xi) SEQUENCE DESCRIPTION: SEQ ID NO:1:
      TTACTCTACT CCCTGAGGAG CTCAGCACAT TTGCC 35
(2) INFORMATION FOR SEQ ID NO:2:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "Primer"
   (xi) SEQUENCE DESCRIPTION: SEQ ID NO:2:
      TATACTTACC AGAATCCGGG CATGGTTCTG GATCA 35